(19) Europäisches Patentamt
European Patent Office
Office européen des brevets

(11) EP 3 885 454 A1

(12) **EUROPEAN PATENT APPLICATION**

(43) Date of publication:
**29.09.2021 Bulletin 2021/39**

(51) Int Cl.:
***C12Q 1/6886*** *(2018.01)*

(21) Application number: **20165097.5**

(22) Date of filing: **24.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **STOFFELS, Monique**
  **5656 AE Eindhoven (NL)**
- **HOFFMANN, Ralf Dieter**
  **5656 AE Eindhoven (NL)**
- **VAN LIESHOUT, Ron Martinus Laurentius**
  **5656 AE Eindhoven (NL)**

(74) Representative: **Philips Intellectual Property & Standards**
**High Tech Campus 5**
**5656 AE Eindhoven (NL)**

# (54) PREDICTION OF RADIOTHERAPY RESPONSE FOR PROSTATE CANCER SUBJECT BASED ON IMMUNE CHECKPOINT GENES

(57) The invention relates to a method of predicting a response of a prostate cancer subject to radiotherapy, comprising determining or receiving the result of a determination of a gene expression profile for each of one or more immune checkpoint genes selected from the group consisting of: LAG3, LGALS9, TDO2, TMIGD2, and VTCN1, said gene expression profile(s) being determined in a biological sample obtained from the subject, and determining the prediction of the radiotherapy response based on the gene expression profile(s) for the one or more immune checkpoint genes.

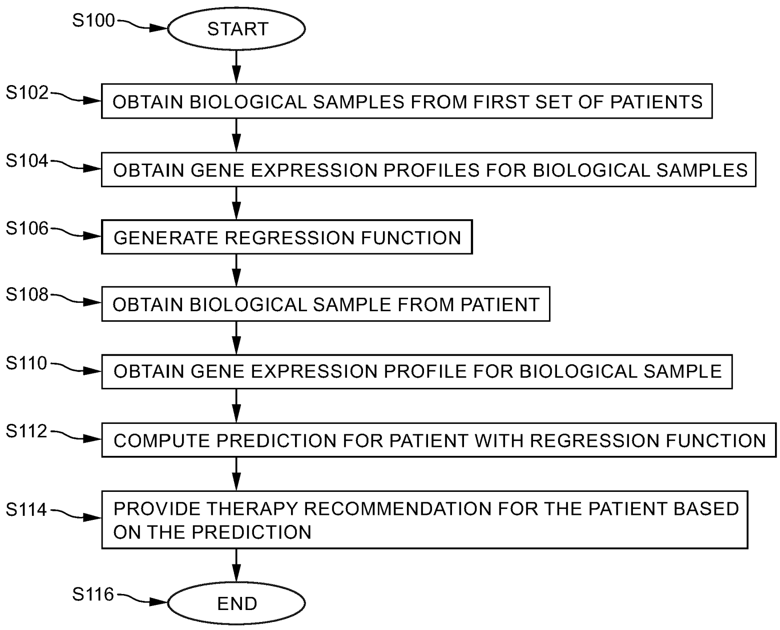


FIG. 1

EP 3 885 454 A1

## Description

FIELD OF THE INVENTION

The invention relates to a method of predicting a response of a prostate cancer subject to radiotherapy. Moreover, the invention relates to a diagnostic kit, to a use of the kit in a method of predicting a response of a prostate cancer subject to radiotherapy, to a use of a gene expression profile for each of one or more immune checkpoint genes in radiotherapy prediction for a prostate cancer subject, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumours, which are self-limited and do not invade or metastasize. Prostate Cancer (PCa) is the second most commonly-occurring non-skin malignancy in men, with an estimated 1.3 million new cases diagnosed and 360,000 deaths world-wide in 2018 (see Bray F. et al., "Global cancer statistics 2018: GLOBOCAN estimates of incidence and mortality worldwide for 36 cancers in 185 countries", CA Cancer J Clin, Vol. 68, No. 6, pages 394-424, 2018). In the US, about 90% of the new cases concern localized cancer, meaning that metastases have not yet been formed (see ACS (American Cancer Society), "Cancer Facts & Figures 2010", 2010).

For the treatment of primary localized prostate cancer, several radical therapies are available, of which surgery (radical prostatectomy, RP) and radiation therapy (RT) are most commonly used. RT is administered via an external beam or via the implantation of radioactive seeds into the prostate (brachytherapy) or a combination of both. It is especially preferable for patients who are not eligible for surgery or have been diagnosed with a tumour in an advanced localized or regional stage. Radical RT is provided to up to 50% of patients diagnosed with localized prostate cancer in the US (see ACS, 2010, ibid).

After treatment, prostate cancer antigen (PSA) levels in the blood are measured for disease monitoring. An increase of the blood PSA level provides a biochemical surrogate measure for cancer recurrence or progression. However, the variation in reported biochemical progression-free survival (bPFS) is large (see Grimm P. et al., "Comparative analysis of prostate-specific antigen free survival outcomes for patients with low, intermediate and high risk prostate cancer treatment by radical therapy. Results from the Prostate Cancer Results Study Group", BJU Int, Suppl. 1, pages 22-29, 2012). For many patients, the bPFS at 5 or even 10 years after radical RT may lie above 90%. Unfortunately, for the group of patients at medium and especially higher risk of recurrence, the bPFS can drop to around 40% at 5 years, depending on the type of RT used (see Grimm P. et al., 2012, ibid).

A large number of the patients with primary localized prostate cancer that are not treated with RT will undergo RP (see ACS, 2010, ibid). After RP, an average of 60% of patients in the highest risk group experience biochemical recurrence after 5 and 10 years (see Grimm P. et al., 2012, ibid). In case of biochemical progression after RP, one of the main challenges is the uncertainty whether this is due to recurring localized disease, one or more metastases or even an indolent disease that will not lead to clinical disease progression (see Dal Pra A. et al., "Contemporary role of postoperative radiotherapy for prostate cancer", Transl Androl Urol, Vo. 7, No. 3, pages 399-413, 2018, and Herrera F.G. and Berthold D.R., "Radiation therapy after radical prostatectomy: Implications for clinicians", Front Oncol, Vol. 6, No. 117, 2016). RT to eradicate remaining cancer cells in the prostate bed is one of the main treatment options to salvage survival after a PSA increase following RP. The effectiveness of salvage radiotherapy (SRT) results in 5-year bPFS for 18% to 90% of patients, depending on multiple factors (see Herrera F.G. and Berthold D.R., 2016, ibid, and Pisansky T.M. et al., "Salvage radiation therapy dose response for biochemical failure of prostate cancer after prostatectomy - A multi-institutional observational study", Int J Radiat Oncol Biol Phys, Vol. 96, No. 5, pages 1046-1053, 2016).

It is clear that for certain patient groups, radical or salvage RT is not effective. Their situation is even worsened by the serious side effects that RT can cause, such as bowel inflammation and dysfunction, urinary incontinence and erectile dysfunction (see Resnick M.J. et al., "Long-term functional outcomes after treatment for localized prostate cancer", N Engl J Med, Vol. 368, No. 5, pages 436-445, 2013, and Hegarty S.E. et al., "Radiation therapy after radical prostatectomy for prostate cancer: Evaluation of complications and influence of radiation timing on outcomes in a large, population-based cohort", PLoS One, Vol. 10, No. 2, 2015). In addition, the median cost of one course of RT based on Medicare reimbursement is $ 18,000, with a wide variation up to about $ 40,000 (see Paravati A.J. et al., "Variation in the cost of radiation therapy among medicare patients with cancer", J Oncol Pract, Vol. 11, No. 5, pages 403-409, 2015). These figures do not include the considerable longitudinal costs of follow-up care after radical and salvage RT.

An improved prediction of effectiveness of RT for each patient, be it in the radical or the salvage setting, would improve therapy selection and potentially survival. This can be achieved by 1) optimizing RT for those patients where RT is predicted to be effective (e.g., by dose escalation or a different starting time) and 2) guiding patients where RT is predicted not to be effective to an alternative, potentially more effective form of treatment. Further, this would reduce suffering for those patients who would be spared ineffective therapy and would reduce costs spent on ineffective therapies.

Numerous investigations have been conducted into measures for response prediction of radical RT (see Hall W.A. et al., "Biomarkers of outcome in patients with localized prostate cancer treated with radiotherapy", Semin Radiat Oncol, Vol. 27, pages 11-20, 2016, and Raymond E. et al., "An appraisal of analytical tools used in predicting clinical outcomes following radiation therapy treatment of men with prostate cancer: A systematic review", Radiat Oncol, Vol. 12, No. 1, page 56, 2017) and SRT (see Herrera F.G. and Berthold D.R., 2016, ibid). Many of these measures depend on the concentration of the blood-based biomarker PSA. Metrics investigated for prediction of response before start of RT (radical as well as salvage) include the absolute value of the PSA concentration, its absolute value relative to the prostate volume, the absolute increase over a certain time and the doubling time. Other frequently considered factors are the Gleason score and the clinical tumour stage. For the SRT setting, additional factors are relevant, e.g., surgical margin status, time to recurrence after RP, pre-/peri-surgical PSA values and clinico-pathological parameters.

Although these clinical variables provide limited improvements in patient stratification in various risk groups, there is a need for better predictive tools.

A wide range of biomarker candidates in tissue and bodily fluids has been investigated, but validation is often limited and generally demonstrates prognostic information and not a predictive (therapy-specific) value (see Hall W.A. et al., 2016, ibid). A small number of gene expression panels is currently being validated by commercial organizations. One or a few of these may show predictive value for RT in future (see Dal Pra A. et al., 2018, ibid).

In conclusion, a strong need for better prediction of response to RT remains, for primary prostate cancer as well as for the post-surgery setting.

## SUMMARY OF THE INVENTION

It is an objective of the invention to provide a method of predicting a response of a prostate cancer subject to radiotherapy, which allows to make better treatment decisions. It is a further objective of the invention to provide a diagnostic kit, a use of the kit in a method of predicting a response of a prostate cancer subject to radiotherapy, a use of a gene expression profile for each of one or more immune checkpoint genes in radiotherapy prediction for a prostate cancer subject, and a corresponding computer program product.

In a first aspect of the present invention, a method of predicting a response of a prostate cancer subject to radiotherapy is presented, comprising:

- determining or receiving the result of a determination of a gene expression profile for each of one or more, for example, 1, 2, 3, 4 or all, immune checkpoint genes selected from the group consisting of: LAG3, LGALS9, TDO2, TMIGD2, and VTCN1, said gene expression profile(s) being determined in a biological sample obtained from the subject,
- determining the prediction of the radiotherapy response based on the gene expression profile(s) for the one or more immune checkpoint genes, and
- optionally, providing the prediction or a therapy recommendation based on the prediction to a medical caregiver or the subject.

In recent years, the importance of the immune system in cancer inhibition as well as in cancer initiation, promotion and metastasis has become very evident (see Mantovani A. et al., "Cancer-related inflammation", Nature, Vol. 454, No. 7203, pages 436-444, 2008, and Giraldo N.A. et al., "The clinical role of the TME in solid cancer", Br J Cancer, Vol. 120, No. 1, pages 45-53, 2019). The immune cells and the molecules they secrete form a crucial part of the tumour microenvironment and most immune cells can infiltrate the tumour tissue. The immune system and the tumour affect and shape one another. Thus, anti-tumour immunity can prevent tumour formation while an inflammatory tumour environment may promote cancer initiation and proliferation. At the same time, tumour cells that may have originated in an immune system-independent manner will shape the immune microenvironment by recruiting immune cells and can have a pro-inflammatory effect while also suppressing anti-cancer immunity.

Some of the immune cells in the tumour microenvironment will have either a general tumour-promoting or a general tumour-inhibiting effect, while other immune cells exhibit plasticity and show both tumour-promoting and tumour-inhibiting potential. Thus, the overall immune microenvironment of the tumour is a mixture of the various immune cells present, the cytokines they produce and their interactions with tumour cells and with other cells in the tumour microenvironment (see Giraldo N.A. et al., 2019, ibid).

The principles described above with regard to the role of the immune system in cancer in general also apply to prostate cancer. Chronic inflammation has been linked to the formation of benign as well as malignant prostate tissue (see Hall W.A. et al., 2016, ibid) and most prostate cancer tissue samples show immune cell infiltrates. The presence of specific immune cells with a pro-tumour effect has been correlated with worse prognosis, while tumours in which natural killer cells were more activated showed better response to therapy and longer recurrence-free periods (see Shiao S.L. et al., "Regulation of prostate cancer progression by tumor microenvironment", Cancer Lett, Vol. 380, No. 1, pages 340-348, 2016).

While a therapy will be influenced by the immune components of the tumour microenvironment, RT itself extensively affects the make-up of these components (see Barker H.E. et al., "The tumor microenvironment after radiotherapy: Mechanisms of resistance or recurrence", Nat Rev Cancer, Vol. 15, No. 7, pages 409-425, 2015). Because suppressive cell types are comparably radiation-insensitive, their relative numbers will increase. Counteractively, the inflicted radiation damage activates cell survival pathways and stimulates the immune system, triggering inflammatory responses and immune cell recruitment. Whether the net effect will be tumour-promoting or tumour-suppressing is as yet uncertain, but its potential for enhancement of cancer immunotherapies is being investigated.

The present invention is based on the idea that, since the status of the immune system and of the immune microenvironment have an impact on therapy effectiveness, the ability to identify markers predictive for this effect might help to be better able to predict overall RT response.

Immune checkpoint molecules play a central role in the regulation of the immune response. Based on the interaction between and the combination of a receptor and a ligand, downstream signaling can be either stimulatory or inhibitory. Multiple checkpoint molecules are involved in the activation or suppression of T-cells in the TME, which in their turn play a role in for example clearing of tumour cells.

Upon recognition, the immune system builds an immune response to clear tumour cells. Clonal expansion, activation, and localization of T-cells that recognize tumour-specific antigens presented on the surface of tumour cells are crucial for tumour cell elimination. Once completed, this response must be dampened to prevent collateral damage to surrounding tissue. Immune checkpoints are crucial for self-tolerance, a way in which the immune system is prevented from attacking cells indiscriminately.

Depending on the type immune checkpoint interaction, downstream signaling may lead to promotion or inhibition of T-cell activation. A single T-cell expresses multiple receptors (and ligands) that can form an immune checkpoint with a ligand on another cell (TABLES 1a and 1b), and the balance of overall positive and negative downstream signals will dictate whether a T-cell will be inhibited in its tumour killing function or not.

Tumour cells often express inhibitory checkpoint molecules to evade elimination by the immune system. Therefore, inhibitory checkpoint molecules are targeted in cancer immunotherapies for several types of cancer. RT can modulate the anticancer immune response, including the abscopal effect, and it is recognized that presence and function of the infiltrate can be used to predict therapy outcome. Immune checkpoint inhibition therapy is already applied in several cancer types, and also for prostate cancer clinical relevance is under investigation. For example, although known as a bad marker for immune therapy response, protein expression of PDL1 has been correlated with high risk prostate cancer, and is also an independent biomarker in the prognosis of high-risk prostate cancer received AHT after RP (see Li H. et al., "The immune checkpoint regulator PDL1 is an independent prognostic biomarker for biochemical recurrence in prostate cancer patients following adjuvant hormonal therapy", J Cancer, Vol. 10, No. 14, pages 3102-3111, 2019). Nevertheless, as described in a recent meta-analysis, the field of prostate cancer, despite being a leader in employing clinical tumour immunology tools, has fallen behind in providing solid proofs of success for clinical impact of immune checkpoint inhibitors in metastatic castrate resistant prostate cancer (see Taghizadeh H. et al., "Immune checkpoint inhibitors in mCRPC - Rationales, challenges and perspectives", Oncoimmunology, Vol. 8, No. 11, 2019). Therefore, although suggestive, it is extremely difficult to conclude based on literature which members of the immune checkpoint molecules might be specifically predictive for the response of prostate cancer patients to radical RT or SRT.

The identified immune checkpoint genes LAG3, LGALS9, TDO2, TMIGD2, and VTCN1 were identified as follows: A group of 538 prostate cancer patients were treated with RP and the prostate cancer tissue was stored. A number of these patients experienced biochemical recurrence and was treated with SRT. For 151 of these patients, the RNA expressed in the originally stored prostate cancer tissue was analysed using RNA sequencing. The mRNA expression of immune checkpoint related genes was compared for the 26 out of 151 patients that died due to prostate cancer, versus the 125 out of 151 patients who survived. For the five molecules LAG3, LGALS9, TDO2, TMIGD2, and VTCN1, the expression level was significantly different for the survivors, suggesting that they have value in the prediction of survival after SRT. Several of these five molecules were found to correlate with prostate cancer, be it in different settings, as described further below.

The term "LAG3" refers to the Lymphocyte-Activation gene 3 (Ensembl: ENSG00000089692), for example, to the sequence as defined in NCBI Reference Sequence NM_002286, specifically, to the nucleotide sequence as set forth in SEQ ID NO:1, which corresponds to the sequence of the above indicated NCBI Reference Sequence of the LAG3 transcript, and also relates to the corresponding amino acid sequence for example as set forth in SEQ ID NO:2, which corresponds to the protein sequence defined in NCBI Protein Accession Reference Sequence NP_002277 encoding the LAG3 polypeptide.

The term "LAG3" also comprises nucleotide sequences showing a high degree of homology to LAG3, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 1 or amino acid sequences being at least 75%, 80%, 85%, 90%, 91%, 92%, 93%, 94%, 95%, 96%, 97%, 98% or 99% identical to the sequence as set forth in SEQ ID NO:2 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:2 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:1.

The term "LGALS9" refers to the Galectin-9 gene (Ensembl: ENSG00000168961), for example, to the sequence as defined in NCBI Reference Sequence NM_002308 or in NCBI Reference Sequence NM_009587 or in NCBI Reference Sequence NM_001330163, specifically, to the nucleotide sequences as set forth in SEQ ID NO:3 or in SEQ ID NO:4 or in SEQ ID NO:5, which correspond to the sequences of the above indicated NCBI Reference Sequences of the LGALS9 transcript, and also relates to the corresponding amino acid sequences for example as set forth in SEQ ID NO:6 or in SEQ ID NO:7 or in SEQ ID NO:8, which correspond to the protein sequences defined in NCBI Protein Accession Reference Sequence NP_002299 and in NCBI Protein Accession Reference Sequence NP_033665 and in NCBI Protein Accession Reference Sequence NP_001317092 encoding the LGALS9 polypeptide.

The term "LGALS9" also comprises nucleotide sequences showing a high degree of homology to LGALS9, e.g., nucleic acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or in SEQ ID NO:7 or in SEQ ID NO:8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or in SEQ ID NO:7 or in SEQ ID NO:8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or in SEQ ID NO:5.

The term "TDO2" refers to the Tryptophan 2,3-Dioxygenase gene (Ensembl: ENSG00000151790), for example, to the sequence as defined in NCBI Reference Sequence NM_005651, specifically, to the nucleotide sequence as set forth in SEQ ID NO:9, which corresponds to the sequence of the above indicated NCBI Reference Sequence of the TDO2 transcript, and also relates to the corresponding amino acid sequence for example as set forth in SEQ ID NO: 10, which corresponds to the protein sequence defined in NCBI Protein Accession Reference Sequence NP_005642 encoding the TDO2 polypeptide.

The term "TDO2" also comprises nucleotide sequences showing a high degree of homology to TDO2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO: 10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:9.

The term "TMIGD2" refers to the Transmembrane and Immunoglobulin Domain Containing 2 gene (Ensembl: ENSG00000167664), for example, to the sequence as defined in NCBI Reference Sequence NM_144615 or in NCBI Reference Sequence NM_001169126 or in NCBI Reference Sequence NM_001308232, specifically, to the nucleotide sequences as set forth in SEQ ID NO:11 or in SEQ ID NO: 12 or in SEQ ID NO: 13, which correspond to the sequences of the above indicated NCBI Reference Sequences of the TMIGD2 transcript, and also relates to the corresponding amino acid sequences for example as set forth in SEQ ID NO:14 or in SEQ ID NO:15 or in SEQ ID NO:16, which correspond to the protein sequences defined in NCBI Protein Accession Reference Sequence NP_653216 and in NCBI Protein Accession Reference Sequence NP_001162597 and in NCBI Protein Accession Reference Sequence NP_001295161 encoding the TMIGD2 polypeptide.

The term "TMIGD2" also comprises nucleotide sequences showing a high degree of homology to TMIGD2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO:12 or in SEQ ID NO:13 or amino acid sequences being at least 75%, 80%, 85%, 90%, 91%, 92%, 93%, 94%, 95%, 96%, 97%, 98% or 99% identical to the sequence as set forth in SEQ ID NO:14 or in SEQ ID NO:15 or in SEQ ID NO:16 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:14 or in SEQ ID NO:15 or in SEQ ID NO:16 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO:12 or in SEQ ID NO:13.

The term "VTCN1" refers to the V-Set Domain Containing T Cell Activation Inhibitor 1 gene (Ensembl: ENSG00000134258), for example, to the sequence as defined in NCBI Reference Sequence NM_001253849 or in NCBI Reference Sequence NM_024626 or in NCBI Reference Sequence NM_001253850, specifically, to the nucleotide sequences as set forth in SEQ ID NO:17 or in SEQ ID NO:18 or in SEQ ID NO:19, which correspond to the sequences of the above indicated NCBI Reference Sequences of the VTCN1 transcript, and also relates to the corresponding amino acid sequences for example as set forth in SEQ ID NO:20 or in SEQ ID NO:21 or in SEQ ID NO:22, which correspond to the protein sequences defined in NCBI Protein Accession Reference Sequence NP_001240778 and in NCBI Protein Accession Reference Sequence NP_078902 and in NCBI Protein Accession Reference Sequence NP_001240779.1 encoding the VTCN1 polypeptide.

The term "VTCN1" also comprises nucleotide sequences showing a high degree of homology to VTCN1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:17 or in SEQ ID NO:18 or in SEQ ID NO:19 or amino acid sequences being at least 75%, 80%, 85%, 90%, 91%, 92%, 93%, 94%, 95%, 96%, 97%, 98% or 99% identical to the sequence as set forth in SEQ ID NO:20 or in SEQ ID NO:21 or in SEQ ID NO:22 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or in SEQ ID NO:21 or in SEQ ID NO:22 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 17 or in SEQ ID NO: 18 or in SEQ ID NO: 19.

The term "biological sample" or "sample obtained from a subject" refers to any biological material obtained via suitable methods known to the person skilled in the art from a subject, e.g., a prostate cancer patient. The biological sample used may be collected in a clinically acceptable manner, e.g., in a way that nucleic acids (in particular RNA) or proteins are preserved.

The biological sample(s) may include body tissue and/or a fluid, such as, but not limited to, blood, sweat, saliva, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the prostate of a male subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., prostate cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

The term "prostate cancer" refers to a cancer of the prostate gland in the male reproductive system, which occurs when cells of the prostate mutate and begin to multiply out of control. Typically, prostate cancer is linked to an elevated level of prostate-specific antigen (PSA). In one embodiment of the present invention the term "prostate cancer" relates to a cancer showing PSA levels above 3.0. In another embodiment the term relates to cancer showing PSA levels above 2.0. The term "PSA level" refers to the concentration of PSA in the blood in ng/ml.

The term "non-progressive prostate cancer state" means that a sample of an individual does not show parameter values indicating "biochemical recurrence" and/or "clinical recurrence" and/or "metastases" and/or "castration-resistant disease" and/or "prostate cancer or disease specific death".

The term "progressive prostate cancer state" means that a sample of an individual shows parameter values indicating "biochemical recurrence" and/or "clinical recurrence" and/or "metastases" and/or "castration-resistant disease" and/or "prostate cancer or disease specific death".

The term "biochemical recurrence" generally refers to recurrent biological values of increased PSA indicating the presence of prostate cancer cells in a sample. However, it is also possible to use other markers that can be used in the detection of the presence or that rise suspicion of such presence.

The term "clinical recurrence" refers to the presence of clinical signs indicating the presence of tumour cells as measured, for example using in vivo imaging. The term "metastases" refers to the presence of metastatic disease in organs other than the prostate.

The term "castration-resistant disease" refers to the presence of hormone-insensitive prostate cancer; i.e., a cancer in the prostate that does not any longer respond to androgen deprivation therapy (ADT).

The term "prostate cancer specific death or disease specific death" refers to death of a patient from his prostate cancer.

It is preferred that the one or more immune checkpoint genes comprise three or more, preferably, all of the immune checkpoint genes.

It is preferred that the determining of the prediction of the radiotherapy response comprises combining the gene expression profiles for two or more, for example, 2, 3, 4 or all, of the immune checkpoint genes with a regression function that had been derived from a population of prostate cancer subjects.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t)=H_0(t) \cdot \exp(w_1 \cdot V_1 + w_2 \cdot V_2 + w_3 \cdot V_3 + ...)$, where $V_1$, $V_2$, $V_3$ ... are predictor variables and $H_0(t)$ is the baseline hazard while $H(t)$ is the hazard at any time t. The hazard ratio (or the risk to reach the event) is represented by $Ln[H(t)/ H_0(t)]= w_1 \cdot V_1 + w_2 \cdot V_2 + w_3 \cdot V_3 + ...$, where the coefficients or weights $w_1$, $w_2$, $w_3$ ... are estimated by the Cox regression analysis and can be interpreted in a similar manner as for logistic regression analysis.

In one particular realization, the prediction of the radiotherapy response is determined as follows:

$$(w_1 \cdot LAG3) + (w_2 \cdot LGALS9) + (w_3 \cdot TDO2) + (w_4 \cdot TMIGD2) + (w_5 \cdot VTCN1) \quad (1)$$

where $w_1$ to $w_5$ are weights and LAG3, LGALS9, TDO2, TMIGD22, and VTCN1 are the expression levels of the immune checkpoint genes.

In one example, $w_1$ may be about 0.0 to 1.0, such as 0.5539, $w_2$ may be about-0.5 to 0.5, such as -0.1195, $w_3$ may be about 1.0 to 2.0, such as 1.2637, $w_4$ may be about 0.0 to 1.0, such as 0.2684, and $w_5$ may be about 0.0 to 1.0, such as 0.4617.

The prediction of the radiotherapy response may also be classified or categorized into one of at least two risk groups, based on the value of the prediction of the radiotherapy response. For example, there may be two risk groups, or three risk groups, or four risk groups, or more than four predefined risk groups. Each risk group covers a respective range of (non-overlapping) values of the prediction of the radiotherapy response. For example, a risk group may indicate a probability of occurrence of a specific clinical event from 0 to <0.1 or from 0.1 to <0.25 or from 0.25 to <0.5 or from 0.5 to 1.0 or the like.

It is further preferred that the determining of the prediction of the radiotherapy response is further based on one or more clinical parameters obtained from the subject.

As mentioned above, various measures based on clinical parameters have been investigated. By further basing the prediction of the radiotherapy response on such clinical parameter(s), it can be possible to further improve the prediction.

It is preferred that the one or more clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

It is further preferred that the determining of the prediction of the radiotherapy response comprises combining the gene expression profile(s) for the one or more immune checkpoint genes and the one or more clinical parameters obtained from the subject with a regression function that had been derived from a population of prostate cancer subjects. In one particular realization, the prediction of the radiotherapy response is determined as follows:

$$(w_6 \cdot IC_model) + (w_7 \cdot pGGG) \quad (2)$$

where $w_6$ and $w_7$ are weights, IC_model is the above-described regression model based on the expression profiles for the two or more, for example, 2, 3, 4 or all, of the immune checkpoint genes, and pGGG is the pathological Gleason grade group. In one example, $w_6$ may be about 0.0 to 1.0, such as 0.665, and $w_7$ may be about 0.5 to 1.5, such as 0.7983.

It is preferred that the biological sample is obtained from the subject before the start of the radiotherapy. The gene expression profile(s) may be determined in the form of mRNA or protein in tissue of prostate cancer. Alternatively, if the immune checkpoint genes are present in a soluble form, the gene expression profile(s) may be determined in blood.

It is further preferred that the radiotherapy is radical radiotherapy or salvage radiotherapy.

It is preferred that the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy. The degree to which the prediction is negative may determine the degree to which the recommended therapy deviates from the standard form of radiotherapy.

In a further aspect of the present invention, an apparatus for predicting a response of a prostate cancer subject to radiotherapy is presented, comprising:

- an input adapted to receive data indicative of a gene expression profile for each of one or more, for example, 1, 2, 3, 4 or all, immune checkpoint genes selected from the group consisting of: LAG3, LGALS9, TDO2, TMIGD2, and VTCN1, said gene expression profile(s) being determined in a biological sample obtained from the subject,
- a processor adapted to determine the prediction of the radiotherapy response based on the gene expression profile(s) for the one or more immune checkpoint genes, and
- optionally, a providing unit adapted to provide the prediction or a therapy recommendation based on the prediction to a medical caregiver or the subject.

In a further aspect of the present invention, a computer program product is presented comprising instructions which, when the program is executed by a computer, cause the computer to carry out a method comprising:

- receiving data indicative of a gene expression profile for each of one or more, for example, 1, 2, 3, 4 or all, immune checkpoint genes selected from the group consisting of: LAG3, LGALS9, TDO2, TMIGD2, and VTCN1, said gene expression profile(s) being determined in a biological sample obtained from a prostate cancer subject,
- determining the prediction of the radiotherapy response based on the gene expression profile(s) for the one or more immune checkpoint genes, and
- optionally, providing the prediction or a therapy recommendation based on the prediction to a medical caregiver or the subject.

In a further aspect of the present invention, a diagnostic kit is presented, comprising:

- at least one primer and/or probe for determining the gene expression profile for each of one or more, for example, 1, 2, 3, 4 or all, immune checkpoint genes selected from the group consisting of: LAG3, LGALS9, TDO2, TMIGD2, and VTCN1, in a biological sample obtained from the subject, and
- optionally, an apparatus as defined in claim 10 or a computer program product as defined in claim 11.

In a further aspect of the present invention, a use of the kit as defined in claim 12 is presented.

It is preferred that the use as defined in claim 13 is in a method of predicting a response of a prostate cancer subject to radiotherapy.

In a further aspect of the present invention, a method is presented, comprising:

- receiving a biological sample obtained from a prostate cancer subject,
- using the kit as defined in claim 12 to determine a gene expression profile for each of one or more, for example, 1, 2, 3, 4 or all, immune checkpoint genes selected from the group consisting of: LAG3, LGALS9, TDO2, TMIGD2, and VTCN1, in the biological sample obtained from the subject.

In a further aspect of the present invention, a use of a gene expression profile for each of one or more, for example, 1, 2, 3, 4 or all, immune checkpoint genes selected from the group consisting of: LAG3, LGALS9, TDO2, TMIGD2, and VTCN1, in a method of predicting a response of a prostate cancer subject to radiotherapy is presented, comprising:

- determining the prediction of the radiotherapy response based on the gene expression profile(s) for the one or more immune checkpoint genes, and optionally, providing the prediction or a therapy recommendation based on the prediction to a medical caregiver or the subject.

It shall be understood that the method of claim 1, the diagnostic kit of claim 12, the use of the diagnostic kit of claim 13, the method of claim 15, and the use of a gene expression profile(s) of claim 16 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a flowchart of an embodiment of a method of predicting a response of a prostate cancer subject to radiotherapy.
Fig. 2 shows a ROC curve analysis of two predictive models.
Fig. 3 shows a Kaplan-Meier curve analysis of the Immune Checkpoint model (IC_model). The clinical endpoint that was tested was the time to metastases (TTM) after the start of salvage radiation therapy (SRT) due to post-surgical disease recurrence.
Fig. 4 shows a Kaplan-Meier curve analysis of the Immune Checkpoint & pGGG combination model (IC&pGGG_model). The clinical endpoint that was tested was the time to metastases (TTM) after the start of salvage radiation therapy (SRT) due to post-surgical disease recurrence.
Fig. 5 shows a Kaplan-Meier curve analysis of the Immune Checkpoint model (IC_model). The clinical endpoint that was tested was the time to prostate cancer specific death (PCa Death) after the start of the salvage radiation therapy (SRT) due to post-surgical disease recurrence.
Fig. 6 shows a Kaplan-Meier curve of the Immune Checkpoint & pGGG combination model (IC&pGGG_model). The clinical endpoint that was tested was the time to prostate cancer specific death (PCa Death) after the start of salvage radiation therapy (SRT) due to post-surgical disease recurrence.

DETAILED DESCRIPTION OF EMBODIMENTS

**Overview Of Radiotherapy Response Prediction**

Fig. 1 shows schematically and exemplarily a flowchart of an embodiment of a method of predicting a response of a prostate cancer subject to radiotherapy.

The method begins at step S100.

At step S102, a biological sample is obtained from each of a first set of patients (subjects) diagnosed with prostate cancer. Preferably, monitoring prostate cancer has been performed for these prostate cancer patients over a period of time, such as at least one year, or at least two years, or about five years, after obtaining the biological sample.
At step S104, a gene expression profile for each of two or more, for example, 2, 3, 4 or all, immune checkpoint genes selected from the group consisting of: LAG3, LGALS9, TDO2, TMIGD2, and VTCN1, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the two or more immune checkpoint genes.
At step S106, a regression function for assigning a prediction of the radiotherapy response is determined based on the gene expression profiles for the two or more immune checkpoint genes, LAG3, LGALS9, TDO2, TMIGD2, and/or VTCN1, obtained for at least some of the biological samples obtained for the first set of patients and respective results obtained from the monitoring. In one particular realization, the regression function is determined as specified in Eq. (1) above.
At step S108, a biological sample is obtained from a patient (subject or individual). The patient can be a new patient or one of the first set.
At step S110, a gene expression profile is obtained for each of the two or more, for example, 2, 3, 4 or all, immune checkpoint genes, e.g., by performing PCR on the biological sample.
At step S112, a prediction of the radiotherapy response based on the gene expression profiles for the two or more immune checkpoint genes is determined for the patient using the regression function. This will be described in more detail later in the description.
At S114, a therapy recommendation may be provided, e.g., to the patient or his or her guardian, to a doctor, or to another healthcare worker, based on the prediction. To this end, the prediction may be categorized into one of a predefined set of risk groups, based on the value of the prediction. In one particular realization, the prediction of the radiotherapy response may be negative or positive for the effectiveness of the radiotherapy. If the prediction is negative, the recommended therapy may comprise one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

The method ends at S116.

In one embodiment, the gene expression profiles at steps S104 and S110 are determined by detecting mRNA expression using two or more primers and/or probes and/or two or more sets thereof.

In one embodiment, steps S104 and S110 further comprise obtaining one or more clinical parameters from the first set of patients and the patient, respectively. The one or more clinical parameters may comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score. The regression function for assigning the prediction of the radiotherapy response that is determined in step S106 is then further based on the one or more clinical parameters obtained from at least some of the first set of patients. In step S112, the prediction of the radiotherapy response is then further based on the one or more clinical parameters, e.g., the pathological Gleason grade group (pGGG), obtained from the patient and is determined for the patient using the regression function.

The immune system interacts in a strong manner with prostate cancer, both on a systemic level and in the tumour microenvironment. Immune checkpoint genes play a central role in the regulation of immune activity. Immune checkpoint genes may therefore provide information on the effectiveness of RT. However, which immune checkpoint genes may have predictive value in this application is extremely difficult to deduce from existing literature due to the many factors that influence the exact function of immune checkpoint genes.

We investigated the extent to which the expression of immune checkpoint genes in prostate cancer tissue correlates with the recurrence of disease after radical RT or SRT.

Of the initial list with immune checkpoint related genes (TABLES 1a and 1b), we have identified five members for which the degree of expression in prostate cancer tissue significantly correlates with mortality after SRT, in a cohort of 151 prostate cancer patients (TABLE 2). In addition, four of the five genes showed a significant correlation with the occurrence of metastases after SRT.

TABLE 1a: Immune checkpoint related genes, expressed on T-Cells, included in analysis.

| Gene | Ensemble | Synonyms |
|---|---|---|
| PDCD1 | ENSG00000188389 | CD279, **PD-1,** PD1, SLEB2, Hsle1 |
| CTLA4 | ENSG00000163599 | CD, CD152, CELIAC3, **CTLA-4,** GSE, IDDM12 |
| CD28 | ENSG00000178562 | **CD28** |
| ICOS | ENSG00000163600 | **ICOS,** AILIM, CD278 |
| TMIGD2 | ENSG00000167664 | **TMIGD2,** CD28H, IGPR-1, MGC23244 |
| BTLA | ENSG00000186265 | **BTLA1,** CD272 |
| CD160 | ENSG00000117281 | CD160, BY55, NK1, NK28 |
| TNFRSF9 | ENSG00000049249 | **4-1BB,** CD137, ILA |
| TNFRSF4 | ENSG00000186827 | ACT35, CD134, **OX40,** TXGP1L |
| CD27 | ENSG00000139193 | **CD27,** S152, TNFRSF7, Tp55 |
| CD40LG | ENSG00000102245 | CD154, **CD40L,** HIGM1, IMD3, TNFSF5, TRAP, gp39, hCD40L |
| TNFRSF18 | ENSG00000186891 | AITR, CD357, **GITR** |
| CD96 | ENSG00000153283 | **CD96,** TACTILE |
| CD226 | ENSG00000150637 | CD226, DNAM-1, **DNAM1,** PTA1, TLiSA1 |
| TIGIT | ENSG00000181847 | **TIGIT,** DKFZp667A205, FLJ39873, VSIG9, VSTM3 |
| PVRIG | ENSG00000213413 | **PVRIG,** C7orf15, CD112R, MGC2463 |
| CD200R1 | ENSG00000163606 | **CD200R,** HCRTR2, MOX2R, OX2R |
| CD244 | ENSG00000122223 | **2B4,** NAIL, NKR2B4, Nmrk, SLAMF4, CD244 |
| HAVCR2 | ENSG00000135077 | HAVCR2, CD366, FLJ14428, **TIM3,** TIMD3, Tim-3 |

(continued)

| Gene | Ensemble | Synonyms |
|---|---|---|
| ADORA2A | ENSG00000128271 | ADORA2, RDC8 |
| TNFRSF8 | ENSG00000120949 | CD30, D1S166E, KI-1 |
| SIRPA | ENSG00000198053 | SIRPα, BIT, CD172a, MFR, MYD-1, P84, PTPNS1, SHPS-1, SHPS1, SIRP, SIRP-ALPHA-1, SIRPalpha, SIRPalpha2 |
| LAG3 | ENSG00000089692 | LAG3, CD223 |
| CEACAM1 | ENSG00000079385 | CEACAM1, BGP, BGP1, CD66a |

TABLE 1b: Immune checkpoint related genes, expressed on tumour cells and APCs, included in analysis.

| Gene | Ensemble | Synonyms |
|---|---|---|
| CD274 | ENSG00000120217 | B7-H, B7-H1, B7H1, **PD-L1,** PDCD1LG1, PDL1, CD274 |
| PDCD1LG2 | ENSG00000197646 | PDCDILG2, B7-DC, Btdc, CD273, **PD-L2,** PDL2, bA574F11.2 |
| CD80 | ENSG00000121594 | CD80, B7-1, B7.1, CD28LG, CD28LG1 |
| CD86 | ENSG00000114013 | CD86, B7-2, B7.2, CD28LG2 |
| ICOSLG | ENSG00000 160223 | ICOSLG, B7-H2, B7H2, B7RP-1, B7RP1, B7h, CD275, GL50, ICOS-L, **ICOSL,** KIAA0653 |
| CD276 | ENSG00000103855 | CD276, B7-H3, B7H3, B7RP-2 |
| VTCN1 | ENSG00000134258 | VTCN1, B7-H4, B7H4, B7S1, B7x, FLJ22418 |
| VSIR | ENSG00000 107738 | VSIR, B7-H5, B7H5, C10orf54, Dies1, GI24, PD-1H, SISP1, **VISTA** |
| HHLA2 | ENSG00000114455 | HHLA2, B7-H5, B7H7, B7y |
| TNFRSF14 | ENSG00000157873 | ATAR, CD270, HVEA, **HVEM,** LIGHTR, TR2, TNFRSF14 |
| TNFSF9 | ENSG00000125657 | CD137L, TNFSF9, 4-1BB-L, 4-1BBL |
| TNFSF4 | ENSG00000117586 | TNFSF4, CD252, **OX-40L,** TXGP1, gp34 |
| CD70 | ENSG00000125726 | **CD70,** CD27L, CD27LG, TNFSF7 |
| CD40 | ENSG00000101017 | **CD40,** Bp50, TNFRSF5, p50 |
| TNFSF18 | ENSG00000120337 | AITRL, TL6, h**GITRL**, TNFSF18 |
| PVR | ENSG00000073008 | PVR, **CD155,** HVED, NECL5, Necl-5, PVS, Tage4 |
| NECTIN2 | ENSG00000130202 | NECTIN2, **CD112,** HVEB, PRR2, PVRL2, PVRR2 |
| CD200 | ENSG00000091972 | CD200, MOX1, MOX2, MRC, OX-2 |
| CD48 | ENSG00000117091 | CD48, BCM1, BLAST, SLAMF2, hCD48, mCD48 |
| LGALS9 | ENSG00000 168961 | Galectin-9, GAL9, LGALS9, LGALS9A |
| IDO1 | ENSG00000131203 | IDOl, IDO, INDO |
| TDO2 | ENSG00000151790 | TDO2, **TDO,** TPH2 |
| TNFSF8 | ENSG00000106952 | CD153, CD30LG |
| CD47 | ENSG00000196776 | CD47, IAP, MER6, OA3 |
| CD209 | ENSG00000090659 | DC-SIGN, CDSIGN, CLEC4L, DC-SIGN, DC-SIGN1, CD209 |

Based on the significant correlation with outcome after RT, we expect that the identified molecules will provide predictive value with regard to the effectiveness of radical RT and/or SRT.

TABLE 2: Univariate Cox regression analysis in a cohort with 151 prostate cancer patients. The tested endpoints in the patient cohort were a) post-treatment metastases progression free survival, and b) post-treatment disease specific survival for men undergoing salvage radiation (SRT) after post-surgical disease recurrence. The number of events and percentage per endpoint is indicated in parenthesis. The table indicates for each tested immune checkpoint gene the association in terms of risk (Hazard Ratio) and significance (p-value) to the tested endpoint.

| **Data Set** | **(Prostate RNAseq)** | | | |
|---|---|---|---|---|
| **# Patients** | **151** | | | |
| **Outcome** | **Post-Salvage-Radiation Outcome** | | | |
| **Endpoint (# events / # patients; % events)** | **Metastases (#65/#151; 43.0%)** | | **Prostate Cancer Mortality (#26/#151; 17.2%)** | |
| **Immune Checkpoint** | **p-value** | **HR** | **p-value** | **HR** |
| **LAG3** | 0.02 | 1.5 | 0.02 | 1.7 |
| **LGALS9** | 0.002 | 1.6 | 0.05 | 1.6 |
| **TDO2** | 0.1 | 2.3 | 0.003 | 4.5 |
| **TMIGD2** | 0.04 | 1.6 | 0.03 | 1.7 |
| **VTCN1** | 0.007 | 2.1 | 0.03 | 2.1 |

The cell surface molecule Lymphocyte activation gene 3 (LAG3) has diverse biologic effects on T-cell function. It maintains tolerance to self and tumor antigens via direct effects on CD8+ T-cells (see Grosso J.F. et al., "LAG-3 regulates CD8+ T cell accumulation and effector function in murine self- and tumor-tolerance systems", J Clin Invest, Vol. 117, No. 11, pages 3383-3392, 2007, but also on a multitude of other lymphocytes (see Long L. et al., "The promising immune checkpoint LAG-3: From tumor microenvironment to cancer immunotherapy", Genes Cancer, Vol. 9, No. 5-6, pages 176-189, 2018). Increased frequencies of LAG3+ lymphocytes have been described in prostate cancer patients with malignancies (see Norström M.M. et al., "Progression of benign prostatic hyperplasia is associated with pro-inflammatory mediators and chronic activation of prostate-infiltrating lymphocytes", Oncotarget, Vol. 7, No. 17, pages 23581-23593, 2016), and it has been suggested a target for cancer treatment.

Galectin-9, encoded by LGALS9, has been associated with metastasis and immunosuppression. Levels are increased in the endothelium of different tumour types (see Heusschen R. et al., "Endothelial LGALS9 splice variant expression in endothelial cell biology and angiogenesis", Biochim Biophys Acta, Vol. 1842, No. 2, pages 284-292, 2014). One study found increased expression of a family member, galectin 1, during tumour disease progression, which was attributed pro-angiogenic activity in prostate tumours. Contrary, in the same study, expression of Galectin-9 was found to decrease during disease progression (see Laderach D.J. et al., "A unique galectin signature in human prostate cancer progression suggests galectin-1 as a key target for treatment of advanced disease", Cancer Res, Vol. 73, No. 1, pages 86-96, 2013). Galectin-9 induces atypical ubiquitination leading to cell death in PC-3 prostate cancer cells (see Itoh A. et al., "Galectin-9 induces atypical ubiquitination leading to cell death in PC-3 prostate cancer cells", Glycobiology, Vol. 29, No. 1, pages 22-35, 2019). Another study in different cancer types also reported a suggested role for Gal-9 in suppressing metastasis (see Nobumoto A. et al., "Galectin-9 suppresses tumor metastasis by blocking adhesion to endothelium and extracellular matrices", Glycobiology, Vol. 18, No. 9, pages 735-744, 2008). Therefore, explaining the role of galectin-9 in metastasis and cancer-related death in our prostate cancer cohort is not trivial.

IDOI and TDO2 catalyse the production of the AhR agonist KYN. Activation of AhR by KYN contributes to the immunosuppressive tumour microenvironment and supports cancer immune escape (see Cheong J.E. and Sun L., "Targeting the IDO1/TDO2-KYN-AhR pathway for cancer immunotherapy - Challenges and opportunities", Trends Pharmacol Sci, Vol. 39, No. 3, pages 307-325, 2018). TDO2 expression has been found in multiple human cancers (see Pilotte L. et al., "Reversal of tumoral immune resistance by inhibition of tryptophan 2,3-dioxygenase", Proc Natl Acad Sci U S A, Vol. 109, No. 7, pages 2497-2502, 2012). In a study comparing prostate cancer samples from young and older men, TDO2 was one of the genes identified with a higher expression in younger patients, and a role in inflammation-induced immune-suppression was suggested (see Ding Y. et al., "Gene expression differences in prostate cancers between young and old men", PLoS Genet, Vol. 12, No. 12, 2016).

TMIGD2 is widely expressed on the plasma membrane of endothelial and epithelial cells and contributes to cell adhesion and migration (see Rahimi N. et al., "Identification of IGPR-1 as a novel adhesion molecule involved in angiogenesis", Molec Biol Cell, Vol. 23, No. 9, pages 1646-1656, 2012). It is also expressed on lymphocytes. HHLA2, which is constitutively expressed on monocytes and induced on B-cells, was identified to bind TMIGD2, and is able to inhibit proliferation and cytokine production of human CD4 and CD8 T-cells in the presence of TCR signaling. HHLA2/TMIGD2 was suggested as a new pathway of the B7/CD28 families representing a novel immunosuppressive mechanism within the tumor microenvironment and an attractive target for human cancer therapy (see Janakiram M. et al., "HHLA2 and TMIGD2: New immunotherapeutic targets of the B7 and CD28 families", Oncoimmunology, Vol. 4, No. 8, 2015).

VTCN1 encodes a protein belonging to the B7 costimulatory protein family, which are present on the surface of antigen-presenting cells and interact with ligands bound to receptors on the surface of T-cells. The potential clinical significance of VTCN1 is supported by the high expression levels found in numerous tumour tissues, including prostate cancer, and correlation with adverse clinical and pathologic features (see Podojil J.R. and Miller S.D., "Potential targeting of B7-H4 for the treatment of cancer", Immunol Rev, Vol. 276, No. 1, pages 40-51, 2017, and MacGregor H.L. and Ohashi P.S., "Molecular pathways: Evaluating the potential for B7-H4 as an immunoregulatory target", Clin Cancer Res, Vol. 23, No. 12, pages 2934-2941, 2017). VTCN1 may initiate dysfunction of tumour-infiltrating CD8+ T cells and may be targeted for cancer immunotherapy (see Li J. et al., "Co-inhibitory molecule B7 superfamily member 1 expressed by tumor-infiltrating myeloid cells induces dysfunction of anti-tumor CD8+ T cells", Immunity, Vol. 48, No. 4, pages 773-786, 2018).

RESULTS

**Cox Regression Analysis**

We then set out to test whether the combination of these five immune checkpoint genes will exhibit more prognostic value. With Cox regression we modelled the expression levels of the five immune checkpoint genes to prostate cancer specific death after post-surgical salvage RT either with (IC&pGGG_model) or without (IC_model) the presence of the variable pathological Gleason grade group (pGGG). We tested the two models in ROC curve analysis as well as in Kaplan-Meier survival analysis.

The Cox regression functions were derived as follows:

IC_model:

$$(w_1 \cdot \mathrm{LAG3}) + (w_2 \cdot \mathrm{LGALS9}) + (w_3 \cdot \mathrm{TDO2}) + (w_4 \cdot \mathrm{TMIGD2}) + (w_5 \cdot \mathrm{VTCN1})$$

IC&pGGG_model:

$$(w_6 \cdot \mathrm{IC_model}) + (w_7 \cdot \mathrm{pGGG})$$

The details for the weights $w_1$ to $w_7$ are shown in the following TABLE 3.

TABLE 3: Variables and weights for the two Cox regression models, i.e., the Immune Checkpoint model (IC_model) and the Immune Checkpoint & pGGG combination model (IC&pGGG_model); NA - not available.

| **Variable** | **Weights** | | |
|---|---|---|---|
| **Model** | | **IC_model** | **IC&pGGG_model** |
| **LAG3** | $w_1$ | 0.5539 | NA |
| **LGALS9** | $w_2$ | -0.1195 | NA |
| **TDO2** | $w_3$ | 1.2637 | NA |
| **TMIGD2** | $w_4$ | 0.2684 | NA |
| **VTCN1** | $w_5$ | 0.4617 | NA |
| **IC_model** | $w_6$ | NA | 0.665 |

(continued)

| Variable | Weights | | |
|---|---|---|---|
| Model | | IC_model | IC&pGGG_model |
| pGGG | $w_7$ | NA | 0.7983 |

**ROC Curve Analysis**

Next, we tested the combined Cox regression model as outlined above its power to predict 10-year prostate cancer specific death after start of salvage radiation due to post-surgical disease recurrence. The performance of the model was compared to the clinical risk score CAPRA-S (see Cooperberg M.R. et al., "The CAPRA-S score: A straightforward tool for improved prediction of outcomes after radical prostatectomy", Cancer, Vol. 117, No. 22, pages 5039-5046, 2011).

Fig. 2 shows a ROC curve analysis of two predictive models. The IC&pGGG_model (AUC=0.9) is the Cox regression model based on five immune checkpoint genes and the pathology Gleason grade group (pGGG) information. The CAPRA_S (AUC=0.74) is the clinical CAPRA-S score (Cancer of the Prostate Risk Assessment score).

**Kaplan-Meier Survival Analysis**

For Kaplan-Meier curve analysis, the Cox regression function of the two risk models (IC_model and IC&pGGG_model) was categorized into two sub-cohorts based on a cut-off (see description of figures below). The goal was to create patient classes by separating the classes with the mean risk score as calculated from the IC_model and IC&pGGG_model, respectively.

The patient classes represent an increasing risk to experience the tested clinical endpoints of time to development of metastases (Figs. 3 and 4) or time to prostate cancer specific death (Figs. 5 and 6) since the start of salvage RT for the two created risk models (IC_model; IC&pGGG_model).

Fig. 3 shows a Kaplan-Meier curve analysis of the IC_model. The clinical endpoint that was tested was the time to metastases (TTM) after the start of salvage radiation therapy (SRT) due to post-surgical disease recurrence. Patients were stratified into two cohorts (low vs. high) according to their risk to experience the clinical endpoint as predicted by the IC regression model using the value 2.8 as cut-off (logrank p=0.008; HR=2.3; 95% CI=1.2-4.3). The following supplementary list indicate the number of patients at risk for the IC_model classes analyzed, i.e., the patients at risk at any time interval +20 months after surgery are shown: Low risk: 92, 77, 73, 70, 64, 49, 46, 23, 3, 0; High risk: 43, 34, 30, 28, 20, 18, 17, 6, 0, 0.

Fig. 4 shows a Kaplan-Meier curve analysis of the IC&pGGG_model. The clinical endpoint that was tested was the time to metastases (TTM) after the start of salvage radiation therapy (SRT) due to post-surgical disease recurrence. Patients were stratified into two cohorts (low vs. high) according to their risk to experience the clinical endpoint as predicted by the IC&pGGG regression model using the value 3.86 as cut-off (logrank p<0.0001; HR=6.4; 95% CI=3.6-11.7). The following supplementary lists indicate the number of patients at risk for the IC&pGGG_model classes analyzed, i.e., the patients at risk at any time interval +20 months after surgery are shown: Low risk: 78, 72, 70, 68, 62, 54, 50, 24, 3, 0; High risk: 57, 39, 33, 30, 22, 13, 13, 5, 0, 0.

Fig. 5 shows a Kaplan-Meier curve analysis of the IC_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC regression model using the value 2.8 as cut-off (logrank p=0.01; HR=3.0; 95% CI=1.3-6.9). The following supplementary list indicate the number of patients at risk for the IC_model classes analyzed, i.e., the patients at risk at any time interval +20 months after surgery are shown: Low risk: 102, 98, 88, 79, 70, 54, 49, 27, 5, 2, 0; High risk: 49, 47, 42, 35, 26, 23, 18, 8, 1, 1, 0.

Fig. 6 shows a Kaplan-Meier curve analysis of the IC&pGGG_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC&pGGG regression model using the value 3.86 as cut-off (logrank p<0.0001; HR=8.4; 95% CI=3.7-18.7). The following supplementary lists indicate the number of patients at risk for the IC&pGGG_model classes analyzed, i.e., the patients at risk at any time interval +20 months after surgery are shown: Low risk: 84, 84, 78, 74, 66, 59, 52, 26, 4, 1, 0; High risk: 67, 61, 52, 40, 30, 18, 15, 9, 2, 2, 0.

For example, the Kaplan-Meier analysis as shown in Figs. 3 to 6 demonstrates the presence of different patient risk groups. The risk group of a patient is determined by the probability to suffer from the respective clinical endpoint (metastases, prostate cancer death) as calculated by the risk model IC_model or IC&pGGG_model. Depending on the predicted risk of a patient (i.e., depending on in which risk group 1 or 2 the patient may belong) different types of interventions might be indicated. In the low risk category, standard of care (SOC) which is SRT potentially combined with SADT (salvage androgen deprivation therapy) delivers acceptable long-term oncological control. Dose escalation of the applied RT and/or combination with chemotherapy might provide improved longitudinal outcomes, and can also be considered. This is definitely not the case for the patient group with a high risk to experience any of the relevant outcomes. In this patient group, escalation of intervention may be indicated. Options for escalation are early combination of SRT (considering higher dose regimens), SADT, and chemotherapy. Other options are alternative therapies like immunotherapies (e.g., Sipuleucil-T), especially since the risk is defined by expression of immune checkpoint molecules, or other experimental therapies.

**Discussion**

The effectiveness of both radical RT and SRT for localized prostate cancer is limited, resulting in disease progression and ultimately death of patients, especially for those at high risk of recurrence. The prediction of the therapy outcome is very complicated as many factors play a role in therapy effectiveness and disease recurrence. It is likely that important factors have not yet been identified, while the effect of others cannot be determined precisely. Multiple clinicopathological measures are currently investigated and applied in a clinical setting to improve response prediction and therapy selection, providing some degree of improvement. Nevertheless, a strong need remains for better prediction of the response to radical RT and to SRT, in order to increase the success rate of these therapies.

We have identified molecules of which expression shows a significant relation to mortality after radical RT and SRT and therefore are expected to improve the prediction of the effectiveness of these treatments. An improved prediction of effectiveness of RT for each patient be it in the radical or the salvage setting, will improve therapy selection and potentially survival. This can be achieved by 1) optimizing RT for those patients where RT is predicted to be effective (e.g. by dose escalation or a different starting time) and 2) guiding patients where RT is predicted not to be effective to an alternative, potentially more effective form of treatment. Further, this would reduce suffering for those patients who would be spared ineffective therapy and would reduce cost spent on ineffective therapies.

Other variations to the disclosed realizations can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in Fig. 1, can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

The invention relates to a method of predicting a response of a prostate cancer subject to radiotherapy, comprising determining or receiving the result of a determination of a gene expression profile for each of one or more, for example, 1, 2, 3, 4 or all, immune checkpoint genes selected from the group consisting of: LAG3, LGALS9, TDO2, TMIGD2, and VTCN1, said gene expression profile(s) being determined in a biological sample obtained from the subject,

determining the prediction of the radiotherapy response based on the gene expression profile(s) for the one or more immune checkpoint genes, and optionally, providing the prediction or a therapy recommendation based on the prediction to a medical caregiver or the subject. Since the status of the immune system and of the immune microenvironment have an impact on therapy effectiveness, the ability to identify markers predictive for this effect might help to be better able to predict overall RT response. Immune checkpoint genes play a central role in the regulation of immune activity. The identified immune checkpoint genes were found to exhibit a significant correlation with outcome after RT, wherefore we expect that they will provide predictive value with regard to the effectiveness of radical RT and/or SRT.

**The attached Sequence Listing, entitled 2020PF00064_Sequence Listing_ST25 is incorporated herein by reference, in its entirety.**

**Claims**

1. A method of predicting a response of a prostate cancer subject to radiotherapy, comprising:

    - determining or receiving the result of a determination of a gene expression profile for each of one or more, for example, 1, 2, 3, 4 or all, immune checkpoint genes selected from the group consisting of: LAG3, LGALS9, TDO2, TMIGD2, and VTCN1, said gene expression profile(s) being determined in a biological sample obtained from the subject,
    - determining the prediction of the radiotherapy response based on the gene expression profile(s) for the one or more immune checkpoint genes, and
    - optionally, providing the prediction or a therapy recommendation based on the prediction to a medical caregiver or the subject.

2. The method as defined in claim 1, wherein the one or more immune checkpoint genes comprise three or more, preferably, all of the immune checkpoint genes.

3. The method as defined in claim 1 or 2, wherein the determining of the prediction of the radiotherapy response comprises combining the gene expression profiles for two or more, for example, 2, 3, 4 or all, of the immune checkpoint genes with a regression function that had been derived from a population of prostate cancer subjects.

4. The method as defined in claim 1 or 2, wherein the determining of the prediction of the radiotherapy response is further based on one or more clinical parameters obtained from the subject.

5. The method as defined in claim 4, wherein the clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

6. The method as defined in claim 4 or 5, wherein the determining of the prediction of the radiotherapy response comprises combining the gene expression profile(s) for the one or more immune checkpoint genes and the one or more clinical parameters obtained from the subject are combined with a regression function that had been derived from a population of prostate cancer subjects.

7. The method as defined in any of claims 1 to 6, wherein the biological sample is obtained from the subject before the start of the radiotherapy.

**8.** The method as defined in any of claims 1 to 7, wherein the radiotherapy is radical radiotherapy or salvage radiotherapy.

**9.** The method as defined in any of claims 1 to 8, wherein the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

**10.** An apparatus for predicting a response of a prostate cancer subject to radiotherapy, comprising:

- an input adapted to receive data indicative of a gene expression profile for each of one or more, for example, 1, 2, 3, 4 or all, immune checkpoint genes selected from the group consisting of: LAG3, LGALS9, TDO2, TMIGD2, and VTCN1, said gene expression profile(s) being determined in a biological sample obtained from the subject,
- a processor adapted to determine the prediction of the radiotherapy response based on the gene expression profile(s) for the one or more immune checkpoint genes, and
- optionally, a providing unit adapted to provide the prediction or a therapy recommendation based on the prediction to a medical caregiver or the subject.

**11.** A computer program product comprising instructions which, when the program is executed by a computer, cause the computer to carry out a method comprising:

- receiving data indicative of a gene expression profile for each of one or more, for example, 1, 2, 3, 4 or all, immune checkpoint genes selected from the group consisting of: LAG3, LGALS9, TDO2, TMIGD2, and VTCN1, said gene expression profile(s) being determined in a biological sample obtained from a prostate cancer subject,
- determining the prediction of the radiotherapy response based on the gene expression profile(s) for the one or more immune checkpoint genes, and
- optionally, providing the prediction or a therapy recommendation based on the prediction to a medical caregiver or the subject.

**12.** A diagnostic kit, comprising:

- at least one primer and/or probe for determining the gene expression profile for each of one or more, for example, 1, 2, 3, 4 or all, immune checkpoint genes selected from the group consisting of: LAG3, LGALS9, TDO2, TMIGD2, and VTCN1, in a biological sample obtained from the subject, and
- optionally, an apparatus as defined in claim 10 or a computer program product as defined in claim 11.

**13.** Use of the kit as defined in claim 12.

**14.** The use of the kit as defined in claim 13 in a method of predicting a response of a prostate cancer subject to radiotherapy.

**15.** A method, comprising:

- receiving a biological sample obtained from a prostate cancer subject,
- using the kit as defined in claim 12 to determine a gene expression profile for each of one or more, for example, 1, 2, 3, 4 or all, immune checkpoint genes selected from the group consisting of: LAG3, LGALS9, TDO2, TMIGD2, and VTCN1, in the biological sample obtained from the subject.

**16.** Use of a gene expression profile for each of one or more, for example, 1, 2, 3, 4 or all, immune checkpoint genes selected from the group consisting of: LAG3, LGALS9, TDO2, TMIGD2, and VTCN1, in a method of predicting a response of a prostate cancer subject to radiotherapy, comprising:

- determining the prediction of the radiotherapy response based on the gene expression profile(s) for the one or more immune checkpoint genes, and optionally, providing the prediction or a therapy recommendation based on the prediction to a medical caregiver or the subject.

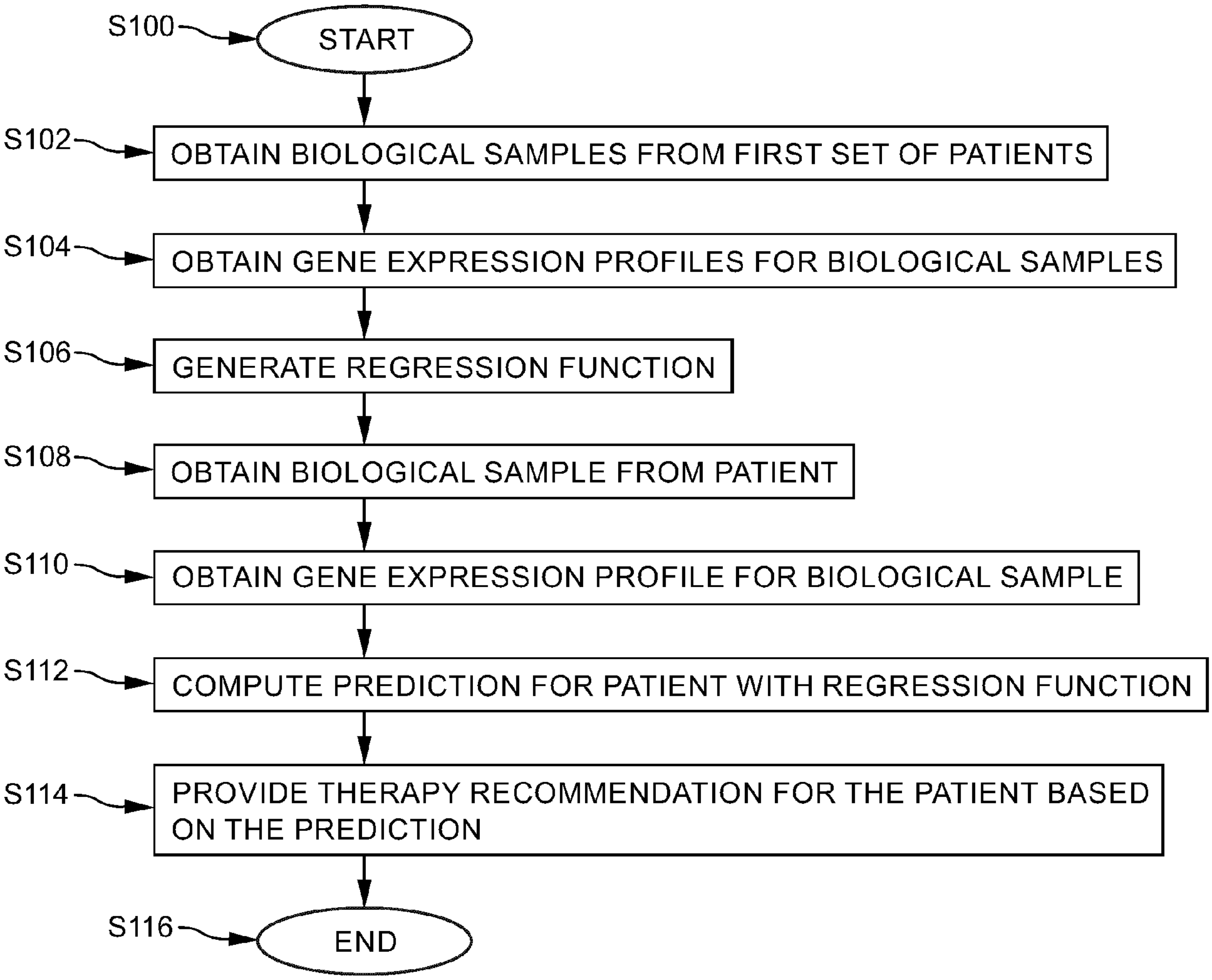
S100
START
S102
OBTAIN BIOLOGICAL SAMPLES FROM FIRST SET OF PATIENTS
S104
OBTAIN GENE EXPRESSION PROFILES FOR BIOLOGICAL SAMPLES
S106
GENERATE REGRESSION FUNCTION
S108
OBTAIN BIOLOGICAL SAMPLE FROM PATIENT
S110
OBTAIN GENE EXPRESSION PROFILE FOR BIOLOGICAL SAMPLE
S112
COMPUTE PREDICTION FOR PATIENT WITH REGRESSION FUNCTION
S114
PROVIDE THERAPY RECOMMENDATION FOR THE PATIENT BASED ON THE PREDICTION
S116
END

FIG. 1

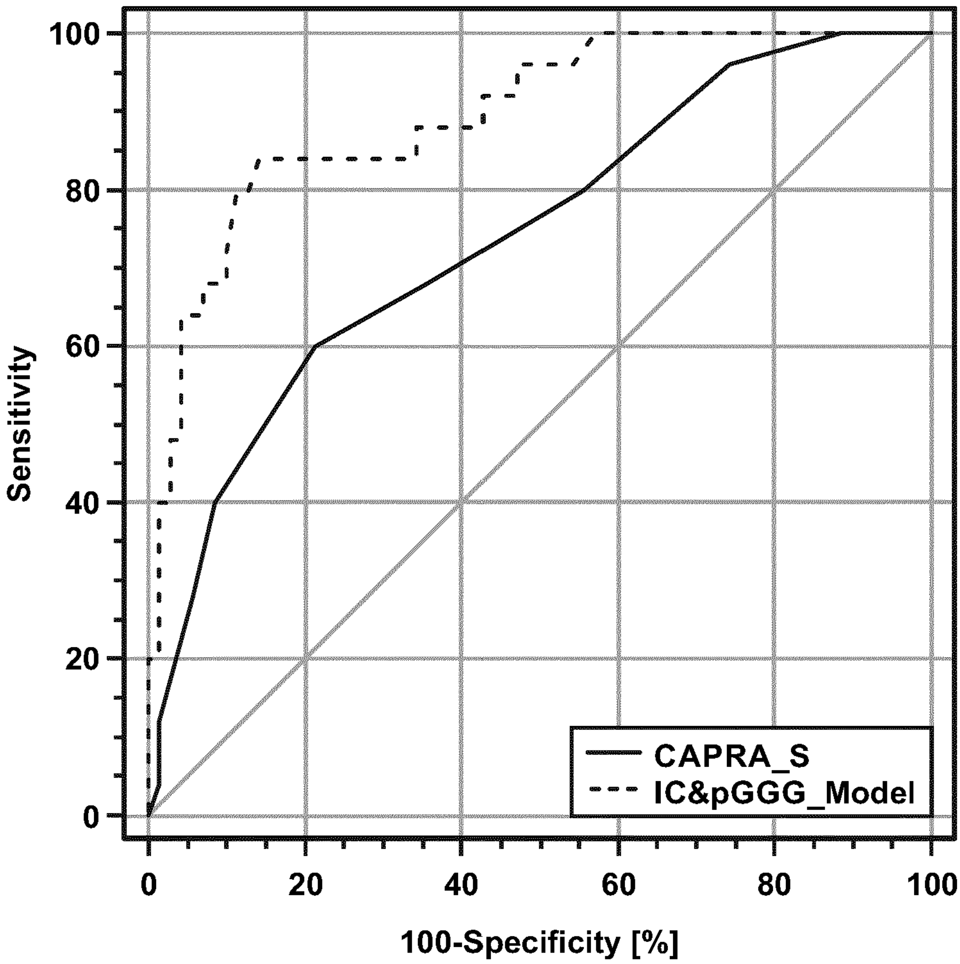
100
80
60
40
20
0
Sensitivity
0
20
40
60
80
100
100-Specificity [%]
CAPRA_S
IC&pGGG_Model

FIG. 2

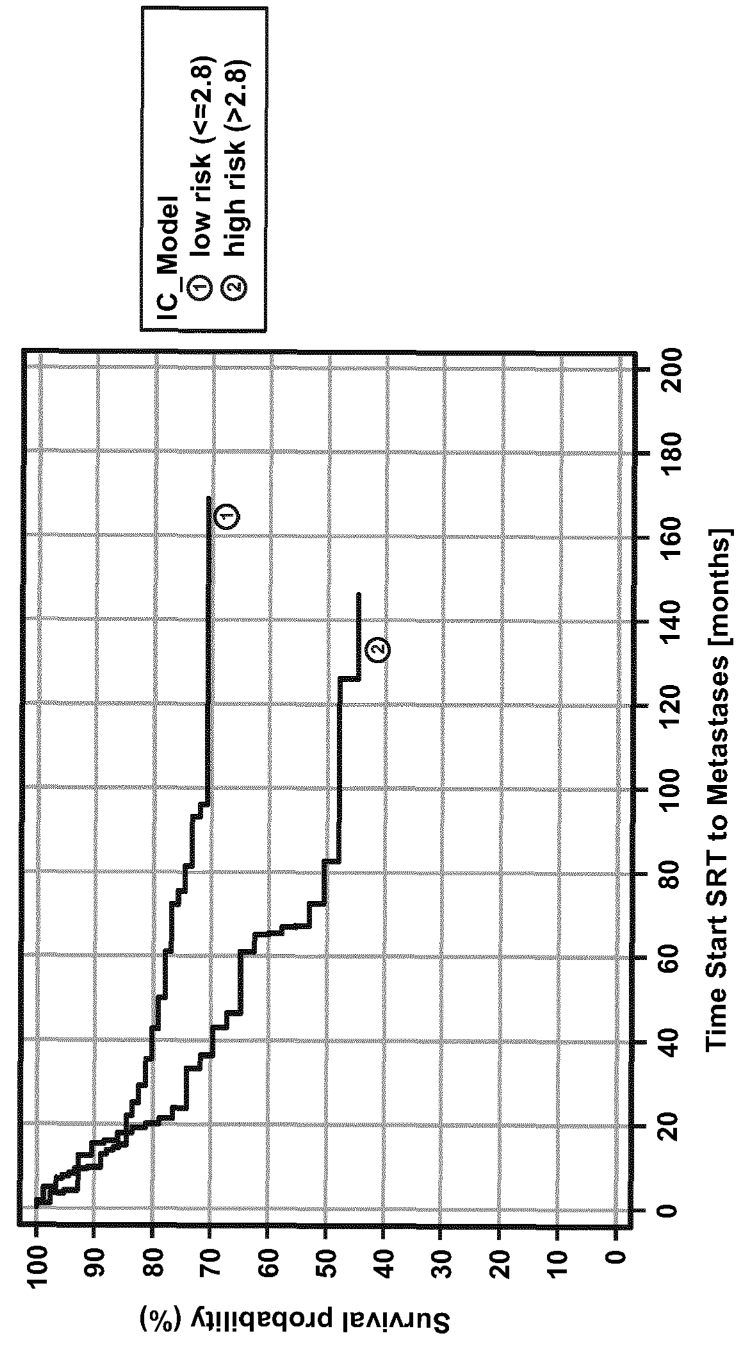
IC_Model
① low risk (<=2.8)
② high risk (>2.8)
Survival probability (%)
100
90
80
70
60
50
40
30
20
10
0
0
20
40
60
80
100
120
140
160
180
200
Time Start SRT to Metastases [months]
①
②

FIG. 3

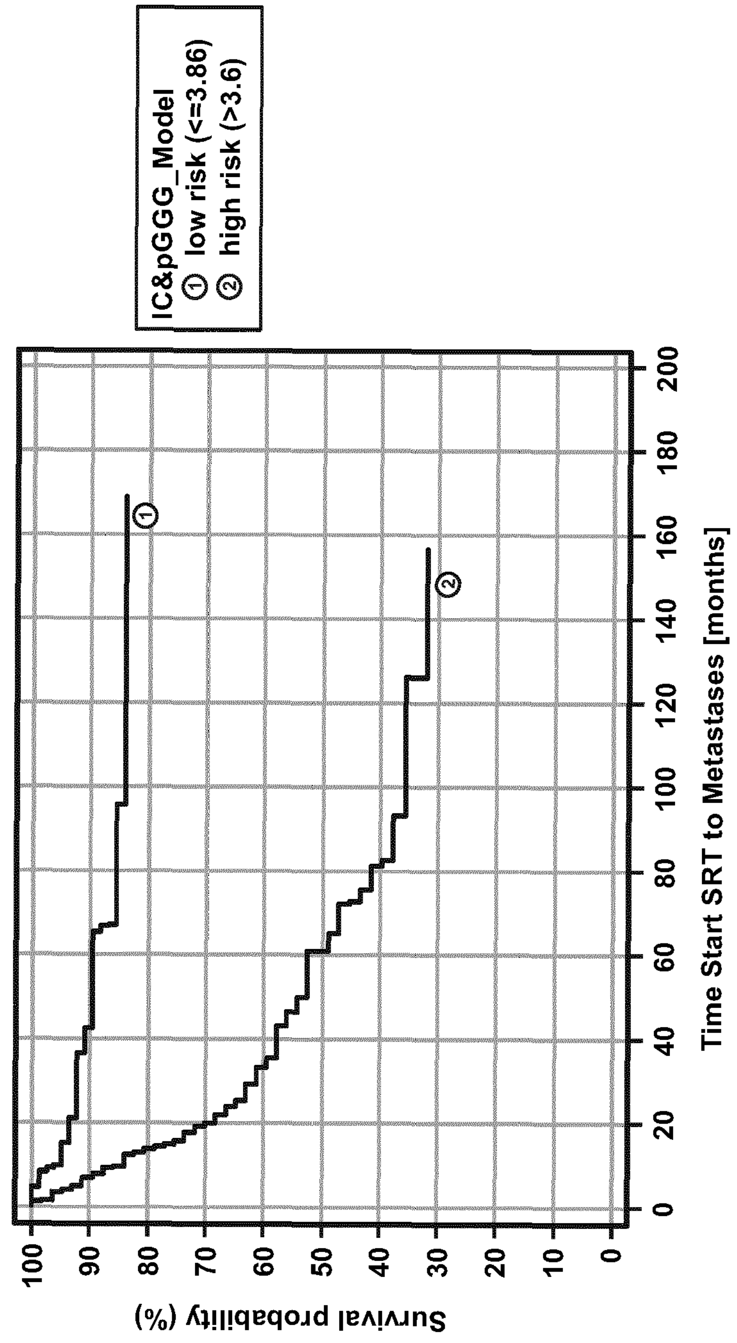
IC&pGGG_Model
① low risk (<=3.86)
② high risk (>3.6)
Survival probability (%)
Time Start SRT to Metastases [months]
100
90
80
70
60
50
40
30
20
10
0
0
20
40
60
80
100
120
140
160
180
200
①
②

FIG. 4

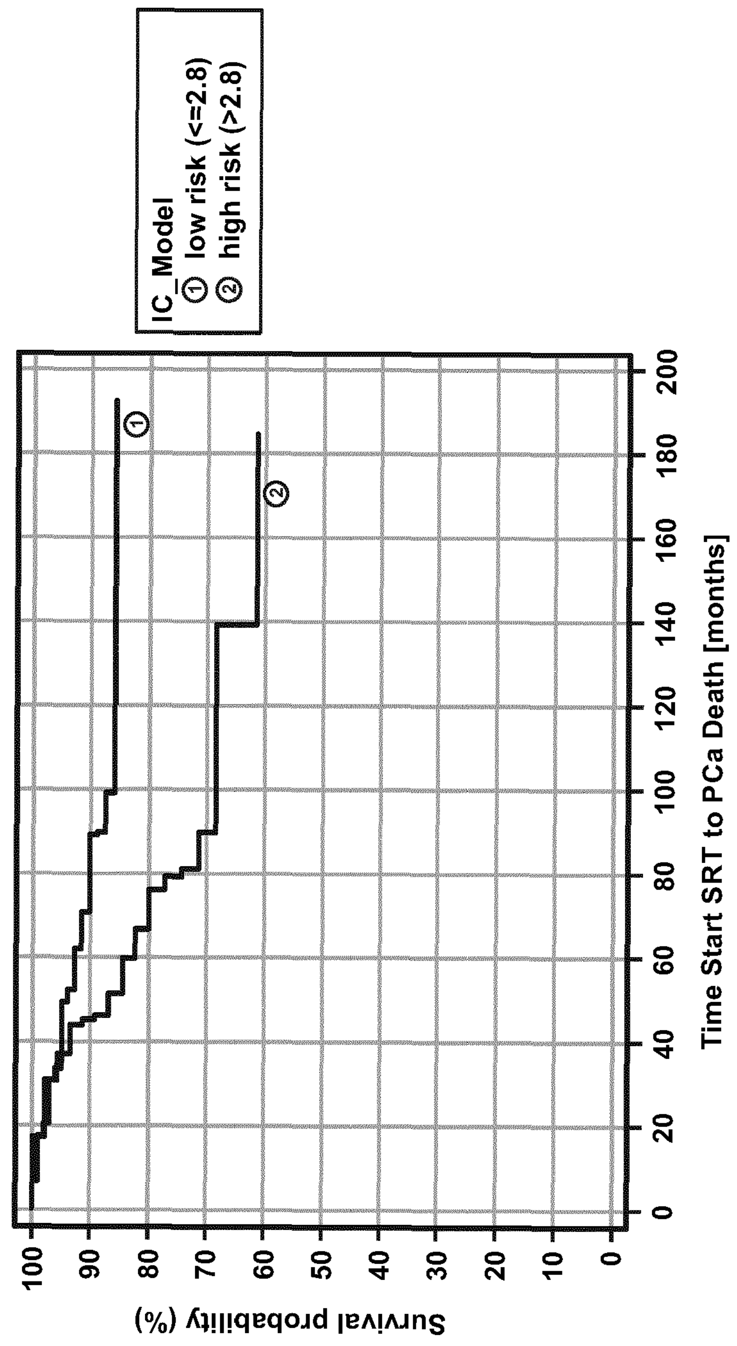
IC_Model
① low risk (<=2.8)
② high risk (>2.8)
Survival probability (%)
Time Start SRT to PCa Death [months]
0
10
20
30
40
50
60
70
80
90
100
120
140
160
180
200
①
②

FIG. 5

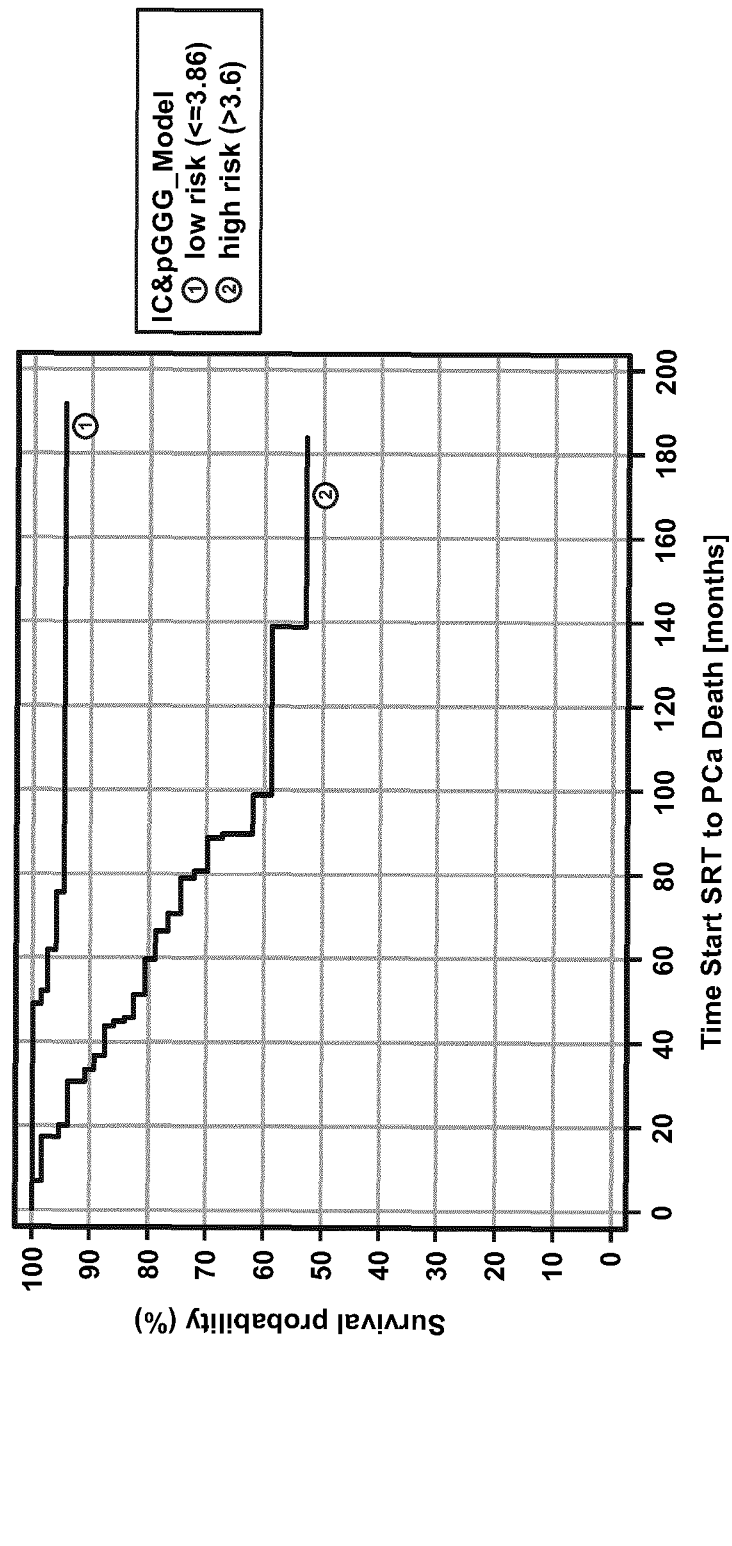
IC&pGGG_Model
① low risk (<=3.86)
② high risk (>3.6)
Survival probability (%)
100
90
80
70
60
50
40
30
20
10
0
0
20
40
60
80
100
120
140
160
180
200
Time Start SRT to PCa Death [months]
①
②

FIG. 6

Europäisches Patentamt
European Patent Office
Office européen des brevets

# EUROPEAN SEARCH REPORT

Application Number
EP 20 16 5097

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "Affymetrix GeneChip Human Genome U133 Array Set HGU133A", NCBI, GEO, Platform GPL96, 11 March 2002 (2002-03-11), pages 1-511, XP055546924, Retrieved from the Internet: URL:https://www.ncbi.nlm.nih.gov/geo/query/acc.cgi?acc=GPL96 [retrieved on 2019-01-24] * the whole document * | 12,13 | INV. C12Q1/6886 |
| Y | ROBERT B. DEN ET AL: "Genomic Classifier Identifies Men With Adverse Pathology After Radical Prostatectomy Who Benefit From Adjuvant Radiation Therapy", JOURNAL OF CLINICAL ONCOLOGY, vol. 33, no. 8, 10 March 2015 (2015-03-10), pages 944-951, XP055592410, US ISSN: 0732-183X, DOI: 10.1200/JCO.2014.59.0026 * the whole document * | 1-11, 14-16 | |
| A | GRASSBERGER CLEMENS ET AL: "Assessing the interactions between radiotherapy and antitumour immunity", NATURE REVIEWS CLINICAL ONCOLOGY, NATURE, NY, US, vol. 16, no. 12, 26 June 2019 (2019-06-26), pages 729-745, XP036928179, ISSN: 1759-4774, DOI: 10.1038/S41571-019-0238-9 [retrieved on 2019-06-26] | 1-16 | TECHNICAL FIELDS SEARCHED (IPC): C12Q |
| Y | WO 2019/028285 A2 (GENOMEDX INC [US]; UNIV MICHIGAN REGENTS [US]) 7 February 2019 (2019-02-07) * the whole document * | 1-11, 14-16 | |

-/--

The present search report has been drawn up for all claims

| Place of search | Date of completion of the search | Examiner |
|---|---|---|
| The Hague | 14 December 2020 | Cornelis, Karen |

4

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 1 of 2

Europäisches Patentamt
European Patent Office
Office européen des brevets

# EUROPEAN SEARCH REPORT

Application Number
EP 20 16 5097

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2017/198617 A1 (DIETRICH DIMO [DE]) 23 November 2017 (2017-11-23)<br>----- | 1-16 | |
| | | | TECHNICAL FIELDS SEARCHED (IPC) |

The present search report has been drawn up for all claims

| Place of search | Date of completion of the search | Examiner |
|---|---|---|
| The Hague | 14 December 2020 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

## ANNEX TO THE EUROPEAN SEARCH REPORT ON EUROPEAN PATENT APPLICATION NO. EP 20 16 5097

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2020

| Patent document cited in search report | Publication date | Patent family member(s) | Publication date |
|---|---|---|---|
| WO 2019028285 A2 | 07-02-2019 | AU 2018310987 A1 | 27-02-2020 |
| | | CA 3072061 A1 | 07-02-2019 |
| | | EP 3662082 A2 | 10-06-2020 |
| | | WO 2019028285 A2 | 07-02-2019 |
| WO 2017198617 A1 | 23-11-2017 | AU 2017267184 A1 | 13-12-2018 |
| | | CN 109715829 A | 03-05-2019 |
| | | DE 102016005947 B3 | 08-06-2017 |
| | | EP 3458602 A1 | 27-03-2019 |
| | | JP 2019516383 A | 20-06-2019 |
| | | US 2019249258 A1 | 15-08-2019 |
| | | WO 2017198617 A1 | 23-11-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BRAY F. et al.** Global cancer statistics 2018: GLOBOCAN estimates of incidence and mortality worldwide for 36 cancers in 185 countries. *CA Cancer J Clin,* 2018, vol. 68 (6), 394-424
- Cancer Facts & Figures 2010. ACS (American Cancer Society), 2010
- CANCER FACTS FIGURES 2010. ACS, 2010
- **GRIMM P. et al.** Comparative analysis of prostate-specific antigen free survival outcomes for patients with low, intermediate and high risk prostate cancer treatment by radical therapy. Results from the Prostate Cancer Results Study Group. *BJU Int,* 2012, vol. 1, 22-29
- **GRIMM P. et al.** *BJU INT,* 2012
- BJU INT. ACS, 2010
- **DAL PRA A. et al.** Contemporary role of postoperative radiotherapy for prostate cancer. *Transl Androl Urol,* 2018, vol. 7 (3), 399-413
- **HERRERA F.G. ; BERTHOLD D.R.** Radiation therapy after radical prostatectomy: Implications for clinicians. *Front Oncol,* 2016, vol. 6 (117
- **HERRERA F.G. ; BERTHOLD D.R.** *FRONT ONCOL,* 2016
- **PISANSKY T.M. et al.** Salvage radiation therapy dose response for biochemical failure of prostate cancer after prostatectomy - A multi-institutional observational study. *Int J Radiat Oncol Biol Phys,* 2016, vol. 96 (5), 1046-1053
- **RESNICK M.J. et al.** Long-term functional outcomes after treatment for localized prostate cancer. *N Engl J Med,* 2013, vol. 368 (5), 436-445
- **HEGARTY S.E. et al.** Radiation therapy after radical prostatectomy for prostate cancer: Evaluation of complications and influence of radiation timing on outcomes in a large, population-based cohort. *PLoS One,* 2015, vol. 10 (2
- **PARAVATI A.J. et al.** Variation in the cost of radiation therapy among medicare patients with cancer. *J Oncol Pract,* 2015, vol. 11 (5), 403-409
- **HALL W.A. et al.** Biomarkers of outcome in patients with localized prostate cancer treated with radiotherapy. *Semin Radiat Oncol,* 2016, vol. 27, 11-20
- **RAYMOND E. et al.** An appraisal of analytical tools used in predicting clinical outcomes following radiation therapy treatment of men with prostate cancer: A systematic review. *Radiat Oncol,* 2017, vol. 12 (1), 56
- **HERRERA F.G. ; BERTHOLD D.R.** *RADIAT ONCOL,* 2016
- **HALL W.A. et al.** *RADIAT ONCOL,* 2016
- **DAL PRA A. et al.** *RADIAT ONCOL,* 2018
- **MANTOVANI A. et al.** Cancer-related inflammation. *Nature,* 2008, vol. 454 (7203), 436-444
- **GIRALDO N.A. et al.** The clinical role of the TME in solid cancer. *Br J Cancer,* 2019, vol. 120 (1), 45-53
- **GIRALDO N.A. et al.** *BR J CANCER,* 2019
- **HALL W.A. et al.** *BR J CANCER,* 2016
- **SHIAO S.L. et al.** Regulation of prostate cancer progression by tumor microenvironment. *Cancer Lett,* 2016, vol. 380 (1), 340-348
- **BARKER H.E. et al.** The tumor microenvironment after radiotherapy: Mechanisms of resistance or recurrence. *Nat Rev Cancer,* 2015, vol. 15 (7), 409-425
- **LI H. et al.** The immune checkpoint regulator PDL1 is an independent prognostic biomarker for biochemical recurrence in prostate cancer patients following adjuvant hormonal therapy. *J Cancer,* 2019, vol. 10 (14), 3102-3111
- **TAGHIZADEH H. et al.** Immune checkpoint inhibitors in mCRPC - Rationales, challenges and perspectives. *Oncoimmunology,* 2019, vol. 8 (11
- **GROSSO J.F. et al.** LAG-3 regulates CD8+ T cell accumulation and effector function in murine self- and tumor-tolerance systems. *J Clin Invest,* 2007, vol. 117 (11), 3383-3392
- **LONG L. et al.** The promising immune checkpoint LAG-3: From tumor microenvironment to cancer immunotherapy. *Genes Cancer,* 2018, vol. 9 (5-6), 176-189
- **NORSTRÖM M.M. et al.** Progression of benign prostatic hyperplasia is associated with pro-inflammatory mediators and chronic activation of prostate-infiltrating lymphocytes. *Oncotarget,* 2016, vol. 7 (17), 23581-23593
- **HEUSSCHEN R. et al.** Endothelial LGALS9 splice variant expression in endothelial cell biology and angiogenesis. *Biochim Biophys Acta,* 2014, vol. 1842 (2), 284-292
- **LADERACH D.J. et al.** A unique galectin signature in human prostate cancer progression suggests galectin-1 as a key target for treatment of advanced disease. *Cancer Res,* 2013, vol. 73 (1), 86-96
- **ITOH A. et al.** Galectin-9 induces atypical ubiquitination leading to cell death in PC-3 prostate cancer cells. *Glycobiology,* 2019, vol. 29 (1), 22-35

- **NOBUMOTO A. et al.** Galectin-9 suppresses tumor metastasis by blocking adhesion to endothelium and extracellular matrices. *Glycobiology,* 2008, vol. 18 (9), 735-744
- **CHEONG J.E. ; SUN L.** Targeting the IDO1/TDO2-KYN-AhR pathway for cancer immunotherapy - Challenges and opportunities. *Trends Pharmacol Sci,* 2018, vol. 39 (3), 307-325
- **PILOTTE L. et al.** Reversal of tumoral immune resistance by inhibition of tryptophan 2,3-dioxygenase. *Proc Natl Acad Sci U S A,* 2012, vol. 109 (7), 2497-2502
- **DING Y. et al.** Gene expression differences in prostate cancers between young and old men. *PLoS Genet,* 2016, vol. 12 (12
- **RAHIMI N. et al.** Identification of IGPR-1 as a novel adhesion molecule involved in angiogenesis. *Molec Biol Cell,* 2012, vol. 23 (9), 1646-1656
- **JANAKIRAM M. et al.** HHLA2 and TMIGD2: New immunotherapeutic targets of the B7 and CD28 families. *Oncoimmunology,* 2015, vol. 4 (8
- **PODOJIL J.R. ; MILLER S.D.** Potential targeting of B7-H4 for the treatment of cancer. *Immunol Rev,* 2017, vol. 276 (1), 40-51
- **MACGREGOR H.L. ; OHASHI P.S.** Molecular pathways: Evaluating the potential for B7-H4 as an immunoregulatory target. *Clin Cancer Res,* 2017, vol. 23 (12), 2934-2941
- **LI J. et al.** Co-inhibitory molecule B7 superfamily member 1 expressed by tumor-infiltrating myeloid cells induces dysfunction of anti-tumor CD8+ T cells. *Immunity,* 2018, vol. 48 (4), 773-786
- **COOPERBERG M.R. et al.** The CAPRA-S score: A straightforward tool for improved prediction of outcomes after radical prostatectomy. *Cancer,* 2011, vol. 117 (22), 5039-5046

SEQUENCE LISTING

<110> Koninklijke Philips N.V.

<120> Prediction of radiotherapy response for prostate cancer subject based on immune checkpoint genes

<130> 2020PF00064

<160> 22

<170> PatentIn version 3.5

<210> 1
<211> 1976
<212> DNA
<213> Homo sapiens

<400> 1
agagaccagc agaacggcat cccagccacg acggccactt tgctctgtct gctctccgcc 60

acggccctgc tctgttccct gggacacccc cgcccccacc tcctcaggct gcctgatctg 120

cccagctttc cagctttcct ctggattccg gcctctggtc atccctcccc accctctctc 180

caaggccctc tcctggtctc ccttcttcta gaacccctte ctccacctcc ctctctgcag 240

aacttctcct ttacccccca ccccccacca ctgcccccctt tccttttctg acctcctttt 300

ggagggctca gcgctgccca gaccatagga gagatgtggg aggctcagtt cctgggcttg 360

ctgtttctgc agccgctttg ggtggctcca gtgaagcctc tccagccagg ggctgaggtc 420

ccggtggtgt gggcccagga gggggctcct gcccagctcc cctgcagccc cacaatcccc 480

ctccaggatc tcagccttct gcgaagagca ggggtcactt ggcagcatca gccagacagt 540

ggcccgcccg ctgccgcccc cggccatccc ctggccccccg gccctcaccc ggcggcgccc 600

tcctcctggg ggcccaggcc ccgccgctac acggtgctga gcgtgggtcc cggaggcctg 660

cgcagcggga ggctgcccct gcagccccgc gtccagctgg atgagcgcgg ccggcagcgc 720

ggggacttct cgctatggct gcgcccagcc cggcgcgcgg acgccggcga gtaccgcgcc 780

gcggtgcacc tcagggaccg cgccctctcc tgccgcctcc gtctgcgcct gggccaggcc 840

tcgatgactg ccagcccccc aggatctctc agagcctccg actgggtcat tttgaactgc 900

tccttcagcc gccctgaccg cccagcctct gtgcattggt tccggaaccg gggccagggc 960

cgagtccctg tccgggagtc cccccatcac cacttagcgg aaagcttcct cttcctgccc 1020

caagtcagcc ccatggactc tgggccctgg ggctgcatcc tcacctacag agatggcttc 1080

aacgtctcca tcatgtataa cctcactgtt ctgggtctgg agcccccaac tcccttgaca 1140

gtgtacgctg gagcaggttc cagggtgggg ctgccctgcc gcctgcctgc tggtgtgggg 1200

acccggtctt tcctcactgc caagtggact cctcctgggg gaggccctga cctcctggtg 1260

actggagaca atggcgactt taccettcga ctagaggatg tgagccaggc ccaggctggg 1320

acctacacct gccatatcca tctgcaggaa cagcagctca atgccactgt cacattggca 1380

atcatcacag tgactcccaa atcctttggg tcacctggat ccctggggaa gctgctttgt 1440

gaggtgactc cagtatctgg acaagaacgc tttgtgtgga gctctctgga caccccatcc 1500

cagaggagtt tctcaggacc ttggctggag gcacaggagg cccagctcct ttcccagcct 1560

tggcaatgcc agctgtacca gggggagagg cttcttggag cagcagtgta cttcacagag 1620

ctgtctagcc caggtgccca acgctctggg agagccccag gtgccctccc agcaggccac 1680

ctcctgctgt ttctcatcct tggtgtcctt tctctgctcc ttttggtgac tggagccttt 1740

ggctttcacc tttggagaag acagtggcga ccaagacgat tttctgcctt agagcaaggg 1800

attcaccctc cgcaggctca gagcaagata gaggagctgg agcaagaacc ggagccggag 1860

ccggagccgg aaccggagcc cgagcccgag cccgagccgg agcagctctg acctggagct 1920

gaggcagcca gcagatctca gcagcccagt ccaaataaac tccctgtcag cagcaa 1976

<210> 2
<211> 525
<212> PRT
<213> Homo sapiens

<400> 2

Met Trp Glu Ala Gln Phe Leu Gly Leu Leu Phe Leu Gln Pro Leu Trp
1               5                   10                  15

Val Ala Pro Val Lys Pro Leu Gln Pro Gly Ala Glu Val Pro Val Val
            20                  25                  30

Trp Ala Gln Glu Gly Ala Pro Ala Gln Leu Pro Cys Ser Pro Thr Ile
        35                  40                  45

Pro Leu Gln Asp Leu Ser Leu Leu Arg Arg Ala Gly Val Thr Trp Gln
    50                  55                  60

His Gln Pro Asp Ser Gly Pro Pro Ala Ala Ala Pro Gly His Pro Leu
65                  70                  75                  80

Ala Pro Gly Pro His Pro Ala Ala Pro Ser Ser Trp Gly Pro Arg Pro
                85                  90                  95

Arg Arg Tyr Thr Val Leu Ser Val Gly Pro Gly Gly Leu Arg Ser Gly
            100                 105                 110

Arg Leu Pro Leu Gln Pro Arg Val Gln Leu Asp Glu Arg Gly Arg Gln
        115                 120                 125

Arg Gly Asp Phe Ser Leu Trp Leu Arg Pro Ala Arg Arg Ala Asp Ala

130 135 140

Gly Glu Tyr Arg Ala Ala Val His Leu Arg Asp Arg Ala Leu Ser Cys
145 150 155 160

Arg Leu Arg Leu Arg Leu Gly Gln Ala Ser Met Thr Ala Ser Pro Pro
165 170 175

Gly Ser Leu Arg Ala Ser Asp Trp Val Ile Leu Asn Cys Ser Phe Ser
180 185 190

Arg Pro Asp Arg Pro Ala Ser Val His Trp Phe Arg Asn Arg Gly Gln
195 200 205

Gly Arg Val Pro Val Arg Glu Ser Pro His His His Leu Ala Glu Ser
210 215 220

Phe Leu Phe Leu Pro Gln Val Ser Pro Met Asp Ser Gly Pro Trp Gly
225 230 235 240

Cys Ile Leu Thr Tyr Arg Asp Gly Phe Asn Val Ser Ile Met Tyr Asn
245 250 255

Leu Thr Val Leu Gly Leu Glu Pro Pro Thr Pro Leu Thr Val Tyr Ala
260 265 270

Gly Ala Gly Ser Arg Val Gly Leu Pro Cys Arg Leu Pro Ala Gly Val
275 280 285

Gly Thr Arg Ser Phe Leu Thr Ala Lys Trp Thr Pro Pro Gly Gly Gly
290 295 300

Pro Asp Leu Leu Val Thr Gly Asp Asn Gly Asp Phe Thr Leu Arg Leu
305 310 315 320

Glu Asp Val Ser Gln Ala Gln Ala Gly Thr Tyr Thr Cys His Ile His
325 330 335

Leu Gln Glu Gln Gln Leu Asn Ala Thr Val Thr Leu Ala Ile Ile Thr
340 345 350

Val Thr Pro Lys Ser Phe Gly Ser Pro Gly Ser Leu Gly Lys Leu Leu
355 360 365

Cys Glu Val Thr Pro Val Ser Gly Gln Glu Arg Phe Val Trp Ser Ser
370 375 380

```
Leu Asp Thr Pro Ser Gln Arg Ser Phe Ser Gly Pro Trp Leu Glu Ala
385                 390                 395                 400

Gln Glu Ala Gln Leu Leu Ser Gln Pro Trp Gln Cys Gln Leu Tyr Gln
                405                 410                 415

Gly Glu Arg Leu Leu Gly Ala Ala Val Tyr Phe Thr Glu Leu Ser Ser
            420                 425                 430

Pro Gly Ala Gln Arg Ser Gly Arg Ala Pro Gly Ala Leu Pro Ala Gly
        435                 440                 445

His Leu Leu Leu Phe Leu Ile Leu Gly Val Leu Ser Leu Leu Leu Leu
    450                 455                 460

Val Thr Gly Ala Phe Gly Phe His Leu Trp Arg Arg Gln Trp Arg Pro
465                 470                 475                 480

Arg Arg Phe Ser Ala Leu Glu Gln Gly Ile His Pro Pro Gln Ala Gln
                485                 490                 495

Ser Lys Ile Glu Glu Leu Glu Gln Glu Pro Glu Pro Glu Pro Glu Pro
            500                 505                 510

Glu Pro Glu Pro Glu Pro Glu Pro Glu Pro Glu Gln Leu
        515                 520                 525


<210>  3
<211>  1628
<212>  DNA
<213>  Homo sapiens

<400>  3
ctttgttaag tcgttccctc tacaaaggac ttcctagtgg gtgtgaaagg cagcggtggc      60

cacagaggcg gcggagagat ggccttcagc ggttcccagg ctccctacct gagtccagct     120

gtcccctttt ctgggactat tcaaggaggt ctccaggacg gacttcagat cactgtcaat     180

gggaccgttc tcagctccag tggaaccagg tttgctgtga actttcagac tggcttcagt     240

ggaaatgaca ttgccttcca cttcaaccct cggtttgaag atggagggta cgtggtgtgc     300

aacacgaggc agaacggaag ctgggggccc gaggagagga agacacacat gcctttccag     360

aaggggatgc cctttgacct ctgcttcctg gtgcagagct cagatttcaa ggtgatggtg     420

aacgggatcc tcttcgtgca gtacttccac cgcgtgccct tccaccgtgt ggacaccatc     480

tccgtcaatg gctctgtgca gctgtcctac atcagcttcc agcctcccgg cgtgtggcct     540

gccaacccgg ctcccattac ccagacagtc atccacacag tgcagagcgc ccctggacag     600

atgttctcta ctcccgccat cccacctatg atgtaccccc accccgccta tccgatgcct     660
```

ttcatcacca ccattctggg agggctgtac ccatccaagt ccatcctcct gtcaggcact 720

gtcctgccca gtgctcagag gttccacatc aacctgtgct ctgggaacca catcgccttc 780

cacctgaacc cccgttttga tgagaatgct gtggtccgca acacccagat cgacaactcc 840

tgggggtctg aggagcgaag tctgccccga aaaatgccct tcgtccgtgg ccagagcttc 900

tcagtgtgga tcttgtgtga agctcactgc ctcaaggtgg ccgtggatgg tcagcacctg 960

tttgaatact accatcgcct gaggaacctg cccaccatca acagactgga agtggggggc 1020

gacatccagc tgacccatgt gcagacatag gcggcttcct ggccctgggg ccgggggctg 1080

gggtgtgggg cagtctgggt cctctcatca tccccacttc ccaggcccag cctttccaac 1140

cctgcctggg atctgggctt taatgcagag gccatgtcct tgtctggtcc tgcttctggc 1200

tacagccacc ctggaacgga gaaggcagct gacggggatt gccttcctca gccgcagcag 1260

cacctggggc tccagctgct ggaatcctac catcccagga ggcaggcaca gccagggaga 1320

ggggaggagt gggcagtgaa gatgaagccc catgctcagt cccctcccat cccccacgca 1380

gctccacccc agtcccaagc caccagctgt ctgctcctgg tgggaggtgg cctcctcagc 1440

ccctcctctc tgacctttaa cctcactctc accttgcacc gtgcaccaac ccttcacccc 1500

tcctggaaag caggcctgat ggcttcccac tggcctccac cacctgacca gagtgttctc 1560

ttcagaggac tggctccttt cccagtgtcc ttaaaataaa gaaatgaaaa tgcttgttgg 1620

cacattca 1628

<210> 4
<211> 1724
<212> DNA
<213> Homo sapiens

<400> 4
ctttgttaag tcgttccctc tacaaaggac ttcctagtgg gtgtgaaagg cagcggtggc 60

cacagaggcg gcggagagat ggccttcagc ggttcccagg ctccctacct gagtccagct 120

gtcccctttt ctgggactat tcaaggaggt ctccaggacg gacttcagat cactgtcaat 180

gggaccgttc tcagctccag tggaaccagg tttgctgtga actttcagac tggcttcagt 240

ggaaatgaca ttgccttcca cttcaaccct cggtttgaag atggagggta cgtggtgtgc 300

aacacgaggc agaacggaag ctgggggccc gaggagagga agacacacat gcctttccag 360

aaggggatgc cctttgacct ctgcttcctg gtgcagagct cagatttcaa ggtgatggtg 420

aacgggatcc tcttcgtgca gtacttccac cgcgtgccct tccaccgtgt ggacaccatc 480

tccgtcaatg gctctgtgca gctgtcctac atcagcttcc agaacccccg cacagtccct 540

gttcagcctg ccttctccac ggtgccgttc tcccagcctg tctgtttccc acccaggccc 600

agggggcgca gacaaaaacc tcccggcgtg tggcctgcca acccggctcc cattacccag 660

acagtcatcc acacagtgca gagcgcccct ggacagatgt tctctactcc cgccatccca 720

cctatgatgt acccccaccc cgcctatccg atgcctttca tcaccaccat tctgggaggg 780

ctgtacccat ccaagtccat cctcctgtca ggcactgtcc tgcccagtgc tcagaggttc 840

cacatcaacc tgtgctctgg gaaccacatc gccttccacc tgaacccccg ttttgatgag 900

aatgctgtgg tccgcaacac ccagatcgac aactcctggg ggtctgagga gcgaagtctg 960

ccccgaaaaa tgcccttcgt ccgtggccag agcttctcag tgtggatctt gtgtgaagct 1020

cactgcctca aggtggccgt ggatggtcag cacctgtttg aatactacca tcgcctgagg 1080

aacctgccca ccatcaacag actggaagtg gggggcgaca tccagctgac ccatgtgcag 1140

acataggcgg cttcctggcc ctggggccgg gggctggggt gtggggcagt ctgggtcctc 1200

tcatcatccc cacttcccag gcccagcctt tccaaccctg cctgggatct gggctttaat 1260

gcagaggcca tgtccttgtc tggtcctgct tctggctaca gccaccctgg aacggagaag 1320

gcagctgacg gggattgcct tcctcagccg cagcagcacc tggggctcca gctgctggaa 1380

tcctaccatc ccaggaggca ggcacagcca gggagagggg aggagtgggc agtgaagatg 1440

aagccccatg ctcagtcccc tcccatcccc cacgcagctc caccccagtc ccaagccacc 1500

agctgtctgc tcctggtggg aggtggcctc ctcagcccct cctctctgac ctttaacctc 1560

actctcacct tgcaccgtgc accaaccctt cacccctcct ggaaagcagg cctgatggct 1620

tcccactggc ctccaccacc tgaccagagt gttctcttca gaggactggc tcctttccca 1680

gtgtccttaa aataaagaaa tgaaaatgct tgttggcaca ttca 1724

<210> 5
<211> 1465
<212> DNA
<213> Homo sapiens

<400> 5
ctttgttaag tcgttccctc tacaaaggac ttcctagtgg gtgtgaaagg cagcggtggc 60

cacagaggcg gcggagagat ggccttcagc ggttcccagg ctccctacct gagtccagct 120

gtcccctttt ctgggactat tcaaggaggt ctccaggacg gacttcagat cactgtcaat 180

gggaccgttc tcagctccag tggaaccagg tttgctgtga actttcagac tggcttcagt 240

ggaaatgaca ttgccttcca cttcaaccct cggtttgaag atggagggta cgtggtgtgc 300

aacacgaggc agaacggaag ctgggggccc gaggagagga agacacacat gcctttccag 360

aaggggatgc cctttgacct ctgcttcctg gtgcagagct cagatttcaa ggtgatggtg 420

aacgggatcc tcttcgtgca gtacttccac cgcgtgccct tccaccgtgt ggacaccatc 480

tccgtcaatg gctctgtgca gctgtcctac atcagcttcc agcctcccgg cgtgtggcct 540

gccaacccgg ctcccattac ccagacagtc atccacacag tgcagagcgc ccctggacag 600

```
atgttctcta ctcccgccat cccacctatg atgtaccccc accccgccta tccgatgcct       660

ttcatcacca ccattctggg agggctgtac ccatccaagt ccatcctcct gtcaggcact       720

gtcctgccca gtgctcagag gtgtggatct tgtgtgaagc tcactgcctc aaggtggccg       780

tggatggtca gcacctgttt gaatactacc atcgcctgag gaacctgccc accatcaaca       840

gactggaagt gggggggcgac atccagctga cccatgtgca gacataggcg gcttcctggc       900

cctggggccg ggggctgggg tgtggggcag tctgggtcct ctcatcatcc ccacttccca       960

ggcccagcct ttccaaccct gcctgggatc tgggctttaa tgcagaggcc atgtccttgt      1020

ctggtcctgc ttctggctac agccaccctg gaacggagaa ggcagctgac ggggattgcc      1080

ttcctcagcc gcagcagcac ctggggctcc agctgctgga atcctaccat cccaggaggc      1140

aggcacagcc agggagaggg gaggagtggg cagtgaagat gaagccccat gctcagtccc      1200

ctcccatccc ccacgcagct ccaccccagt cccaagccac cagctgtctg ctcctggtgg      1260

gaggtggcct cctcagcccc tcctctctga cctttaacct cactctcacc ttgcaccgtg      1320

caccaaccct tcacccctcc tggaaagcag gcctgatggc ttcccactgg cctccaccac      1380

ctgaccagag tgttctcttc agaggactgg ctcctttccc agtgtcctta aaataaagaa      1440

atgaaaatgc ttgttggcac attca                                            1465


<210>  6
<211>  323
<212>  PRT
<213>  Homo sapiens

<400>  6

Met Ala Phe Ser Gly Ser Gln Ala Pro Tyr Leu Ser Pro Ala Val Pro
1               5                   10                  15


Phe Ser Gly Thr Ile Gln Gly Gly Leu Gln Asp Gly Leu Gln Ile Thr
            20                  25                  30


Val Asn Gly Thr Val Leu Ser Ser Ser Gly Thr Arg Phe Ala Val Asn
        35                  40                  45


Phe Gln Thr Gly Phe Ser Gly Asn Asp Ile Ala Phe His Phe Asn Pro
    50                  55                  60


Arg Phe Glu Asp Gly Gly Tyr Val Val Cys Asn Thr Arg Gln Asn Gly
65                  70                  75                  80


Ser Trp Gly Pro Glu Glu Arg Lys Thr His Met Pro Phe Gln Lys Gly
                85                  90                  95
```

Met Pro Phe Asp Leu Cys Phe Leu Val Gln Ser Ser Asp Phe Lys Val
100 105 110

Met Val Asn Gly Ile Leu Phe Val Gln Tyr Phe His Arg Val Pro Phe
115 120 125

His Arg Val Asp Thr Ile Ser Val Asn Gly Ser Val Gln Leu Ser Tyr
130 135 140

Ile Ser Phe Gln Pro Pro Gly Val Trp Pro Ala Asn Pro Ala Pro Ile
145 150 155 160

Thr Gln Thr Val Ile His Thr Val Gln Ser Ala Pro Gly Gln Met Phe
165 170 175

Ser Thr Pro Ala Ile Pro Pro Met Met Tyr Pro His Pro Ala Tyr Pro
180 185 190

Met Pro Phe Ile Thr Thr Ile Leu Gly Gly Leu Tyr Pro Ser Lys Ser
195 200 205

Ile Leu Leu Ser Gly Thr Val Leu Pro Ser Ala Gln Arg Phe His Ile
210 215 220

Asn Leu Cys Ser Gly Asn His Ile Ala Phe His Leu Asn Pro Arg Phe
225 230 235 240

Asp Glu Asn Ala Val Val Arg Asn Thr Gln Ile Asp Asn Ser Trp Gly
245 250 255

Ser Glu Glu Arg Ser Leu Pro Arg Lys Met Pro Phe Val Arg Gly Gln
260 265 270

Ser Phe Ser Val Trp Ile Leu Cys Glu Ala His Cys Leu Lys Val Ala
275 280 285

Val Asp Gly Gln His Leu Phe Glu Tyr Tyr His Arg Leu Arg Asn Leu
290 295 300

Pro Thr Ile Asn Arg Leu Glu Val Gly Gly Asp Ile Gln Leu Thr His
305 310 315 320

Val Gln Thr

<210> 7
<211> 355
<212> PRT

<213> Homo sapiens

<400> 7

Met Ala Phe Ser Gly Ser Gln Ala Pro Tyr Leu Ser Pro Ala Val Pro
1 5 10 15

Phe Ser Gly Thr Ile Gln Gly Gly Leu Gln Asp Gly Leu Gln Ile Thr
20 25 30

Val Asn Gly Thr Val Leu Ser Ser Ser Gly Thr Arg Phe Ala Val Asn
35 40 45

Phe Gln Thr Gly Phe Ser Gly Asn Asp Ile Ala Phe His Phe Asn Pro
50 55 60

Arg Phe Glu Asp Gly Gly Tyr Val Val Cys Asn Thr Arg Gln Asn Gly
65 70 75 80

Ser Trp Gly Pro Glu Glu Arg Lys Thr His Met Pro Phe Gln Lys Gly
85 90 95

Met Pro Phe Asp Leu Cys Phe Leu Val Gln Ser Ser Asp Phe Lys Val
100 105 110

Met Val Asn Gly Ile Leu Phe Val Gln Tyr Phe His Arg Val Pro Phe
115 120 125

His Arg Val Asp Thr Ile Ser Val Asn Gly Ser Val Gln Leu Ser Tyr
130 135 140

Ile Ser Phe Gln Asn Pro Arg Thr Val Pro Val Gln Pro Ala Phe Ser
145 150 155 160

Thr Val Pro Phe Ser Gln Pro Val Cys Phe Pro Pro Arg Pro Arg Gly
165 170 175

Arg Arg Gln Lys Pro Pro Gly Val Trp Pro Ala Asn Pro Ala Pro Ile
180 185 190

Thr Gln Thr Val Ile His Thr Val Gln Ser Ala Pro Gly Gln Met Phe
195 200 205

Ser Thr Pro Ala Ile Pro Pro Met Met Tyr Pro His Pro Ala Tyr Pro
210 215 220

Met Pro Phe Ile Thr Thr Ile Leu Gly Gly Leu Tyr Pro Ser Lys Ser
225 230 235 240

Ile Leu Leu Ser Gly Thr Val Leu Pro Ser Ala Gln Arg Phe His Ile
245 250 255

Asn Leu Cys Ser Gly Asn His Ile Ala Phe His Leu Asn Pro Arg Phe
260 265 270

Asp Glu Asn Ala Val Val Arg Asn Thr Gln Ile Asp Asn Ser Trp Gly
275 280 285

Ser Glu Glu Arg Ser Leu Pro Arg Lys Met Pro Phe Val Arg Gly Gln
290 295 300

Ser Phe Ser Val Trp Ile Leu Cys Glu Ala His Cys Leu Lys Val Ala
305 310 315 320

Val Asp Gly Gln His Leu Phe Glu Tyr Tyr His Arg Leu Arg Asn Leu
325 330 335

Pro Thr Ile Asn Arg Leu Glu Val Gly Gly Asp Ile Gln Leu Thr His
340 345 350

Val Gln Thr
355

<210> 8
<211> 246
<212> PRT
<213> Homo sapiens

<400> 8

Met Ala Phe Ser Gly Ser Gln Ala Pro Tyr Leu Ser Pro Ala Val Pro
1 5 10 15

Phe Ser Gly Thr Ile Gln Gly Gly Leu Gln Asp Gly Leu Gln Ile Thr
20 25 30

Val Asn Gly Thr Val Leu Ser Ser Ser Gly Thr Arg Phe Ala Val Asn
35 40 45

Phe Gln Thr Gly Phe Ser Gly Asn Asp Ile Ala Phe His Phe Asn Pro
50 55 60

Arg Phe Glu Asp Gly Gly Tyr Val Val Cys Asn Thr Arg Gln Asn Gly
65 70 75 80

Ser Trp Gly Pro Glu Glu Arg Lys Thr His Met Pro Phe Gln Lys Gly
85 90 95

Met Pro Phe Asp Leu Cys Phe Leu Val Gln Ser Ser Asp Phe Lys Val
100 105 110

Met Val Asn Gly Ile Leu Phe Val Gln Tyr Phe His Arg Val Pro Phe
115 120 125

His Arg Val Asp Thr Ile Ser Val Asn Gly Ser Val Gln Leu Ser Tyr
130 135 140

Ile Ser Phe Gln Pro Pro Gly Val Trp Pro Ala Asn Pro Ala Pro Ile
145 150 155 160

Thr Gln Thr Val Ile His Thr Val Gln Ser Ala Pro Gly Gln Met Phe
165 170 175

Ser Thr Pro Ala Ile Pro Pro Met Met Tyr Pro His Pro Ala Tyr Pro
180 185 190

Met Pro Phe Ile Thr Thr Ile Leu Gly Gly Leu Tyr Pro Ser Lys Ser
195 200 205

Ile Leu Leu Ser Gly Thr Val Leu Pro Ser Ala Gln Arg Cys Gly Ser
210 215 220

Cys Val Lys Leu Thr Ala Ser Arg Trp Pro Trp Met Val Ser Thr Cys
225 230 235 240

Leu Asn Thr Thr Ile Ala
245

<210> 9
<211> 1700
<212> DNA
<213> Homo sapiens

<400> 9
aggtcaatga tagcatctgc ctagagtcaa acctccgtgc ttctcagaca gtgccttttc 60

accatgagtg ggtgcccatt tttaggaaac aactttggat atacttttaa aaaactcccc 120

gtagaaggca gcgaagaaga caaatcacaa actggtgtga atagagccag caaaggaggt 180

cttatctatg ggaactacct gcatttggaa aaagttttga atgcacaaga actgcaaagt 240

gaaacaaaag gaaataaaat ccatgatgaa catcttttta tcataactca tcaagcttat 300

gaactctggt ttaagcaaat cctctgggag ttggattctg ttcgagagat ctttcagaat 360

ggccatgtca gagatgaaag gaacatgctt aaggttgttt ctcggatgca ccgagtgtca 420

gtgatcctga aactgctggt gcagcagttt tccattctgg agacgatgac agccttggac 480

```
ttcaatgact tcagagagta cttatctcca gcatcaggct tccagagttt gcaattccga      540

ctattagaaa acaagatagg tgttcttcag aacatgagag tcccttataa cagaagacat      600

tatcgtgata acttcaaagg agaagaaaat gaactgctac ttaaatctga gcaggaaaag      660

acacttctgg aattagtgga ggcatggctg gaaagaactc caggtttaga gccacatgga      720

tttaacttct ggggaaagct tgaaaaaaat atcaccagag gcctggaaga ggaattcata      780

aggattcagg ctaaagaaga gtctgaagaa aaagaggaac aggtggctga atttcagaag      840

caaaaagagg tgctactgtc cttatttgat gagaaacgtc atgaacatct ccttagtaaa      900

ggtgaaagac ggctgtcata cagagcactt cagggagcat tgatgatata tttttacagg      960

gaagagccta ggttccaggt gccttttcag ttgctgactt ctcttatgga catagattca     1020

ctgatgacca aatggagata taaccatgtg tgcatggtgc acagaatgct gggcagcaaa     1080

gctggcaccg gtggttcctc aggctatcac tacctgcgat caactgtgag tgataggtac     1140

aaggtatttg tagatttatt taatctttca acatacctga ttccccgaca ctggataccg     1200

aagatgaacc caaccattca caaatttcta tatacagcag aatactgtga tagctcctac     1260

ttcagcagtg atgaatcaga ttaaaatcgt ctgcaaaatc tatgaagaat actggtttca     1320

cagcctattt tttattttct atggattttc ataaatacag tttgaatata tgtatgcata     1380

tattgttcag caccacgatg ctctgattta attctagaaa caatttgatt acctcttgtt     1440

tgtgacaaga ctaagcatta agatgagaaa gaatacattt aaatagtaac attgtacata     1500

gggtgttttc ctattaaaaa ttcagtttcc cctgagactt aatgtaacca cttaatgtaa     1560

tcactatctc attgtttcat ctttataaac ttgtaaactt catctatttc aaatatttta     1620

tgcagtacat tatattattc tgtacaaagg ctttcaaaca aaatttttaa aataataaag     1680

tattaatctt tctccctgta                                                 1700
```

<210> 10
<211> 406
<212> PRT
<213> Homo sapiens

<400> 10

```
Met Ser Gly Cys Pro Phe Leu Gly Asn Asn Phe Gly Tyr Thr Phe Lys
1               5                   10                  15

Lys Leu Pro Val Glu Gly Ser Glu Glu Asp Lys Ser Gln Thr Gly Val
            20                  25                  30

Asn Arg Ala Ser Lys Gly Gly Leu Ile Tyr Gly Asn Tyr Leu His Leu
        35                  40                  45

Glu Lys Val Leu Asn Ala Gln Glu Leu Gln Ser Glu Thr Lys Gly Asn
```

50 55 60

Lys Ile His Asp Glu His Leu Phe Ile Ile Thr His Gln Ala Tyr Glu
65 70 75 80

Leu Trp Phe Lys Gln Ile Leu Trp Glu Leu Asp Ser Val Arg Glu Ile
85 90 95

Phe Gln Asn Gly His Val Arg Asp Glu Arg Asn Met Leu Lys Val Val
100 105 110

Ser Arg Met His Arg Val Ser Val Ile Leu Lys Leu Leu Val Gln Gln
115 120 125

Phe Ser Ile Leu Glu Thr Met Thr Ala Leu Asp Phe Asn Asp Phe Arg
130 135 140

Glu Tyr Leu Ser Pro Ala Ser Gly Phe Gln Ser Leu Gln Phe Arg Leu
145 150 155 160

Leu Glu Asn Lys Ile Gly Val Leu Gln Asn Met Arg Val Pro Tyr Asn
165 170 175

Arg Arg His Tyr Arg Asp Asn Phe Lys Gly Glu Glu Asn Glu Leu Leu
180 185 190

Leu Lys Ser Glu Gln Glu Lys Thr Leu Leu Glu Leu Val Glu Ala Trp
195 200 205

Leu Glu Arg Thr Pro Gly Leu Glu Pro His Gly Phe Asn Phe Trp Gly
210 215 220

Lys Leu Glu Lys Asn Ile Thr Arg Gly Leu Glu Glu Glu Phe Ile Arg
225 230 235 240

Ile Gln Ala Lys Glu Glu Ser Glu Glu Lys Glu Glu Gln Val Ala Glu
245 250 255

Phe Gln Lys Gln Lys Glu Val Leu Leu Ser Leu Phe Asp Glu Lys Arg
260 265 270

His Glu His Leu Leu Ser Lys Gly Glu Arg Arg Leu Ser Tyr Arg Ala
275 280 285

Leu Gln Gly Ala Leu Met Ile Tyr Phe Tyr Arg Glu Glu Pro Arg Phe
290 295 300

Gln Val Pro Phe Gln Leu Leu Thr Ser Leu Met Asp Ile Asp Ser Leu
305 310 315 320

Met Thr Lys Trp Arg Tyr Asn His Val Cys Met Val His Arg Met Leu
325 330 335

Gly Ser Lys Ala Gly Thr Gly Gly Ser Ser Gly Tyr His Tyr Leu Arg
340 345 350

Ser Thr Val Ser Asp Arg Tyr Lys Val Phe Val Asp Leu Phe Asn Leu
355 360 365

Ser Thr Tyr Leu Ile Pro Arg His Trp Ile Pro Lys Met Asn Pro Thr
370 375 380

Ile His Lys Phe Leu Tyr Thr Ala Glu Tyr Cys Asp Ser Ser Tyr Phe
385 390 395 400

Ser Ser Asp Glu Ser Asp
405

<210> 11
<211> 1282
<212> DNA
<213> Homo sapiens

<400> 11
ggaagtctgt caactgggag ggggagaggg gggtgatggg ccaggaatgg ggtccccggg 60

catggtgctg ggcctcctgg tgcagatctg ggccctgcaa gaagcctcaa gcctgagcgt 120

gcagcagggg cccaacttgc tgcaggtgag gcagggcagt caggcgaccc tggtctgcca 180

ggtggaccag gccacagcct gggaacggct ccgtgttaag tggacaaagg atggggccat 240

cctgtgtcaa ccgtacatca ccaacggcag cctcagcctg gggggtctgcg ggccccaggg 300

acggctctcc tggcaggcac ccagccatct caccctgcag ctggaccctg tgagcctcaa 360

ccacagcggg gcgtacgtgt gctgggcggc cgtagagatt cctgagttgg aggaggctga 420

gggcaacata acaaggctct ttgtggaccc agatgacccc acacagaaca gaaaccggat 480

cgcaagcttc ccaggattcc tcttcgtgct gctgggggtg ggaagcatgg gtgtggctgc 540

gatcgtgtgg ggtgcctggt tctggggccg ccgcagctgc cagcaaaggg actcaggtaa 600

cagcccagga aatgcattct acagcaacgt cctataccgg ccccgggggg ccccaaagaa 660

gagtgaggac tgctctggag aggggaagga ccagaggggc cagagcattt attcaacctc 720

cttcccgcaa ccggcccccc gccagccgca cctggcgtca agaccctgcc ccagcccgag 780

accctgcccc agccccaggc ccggccaccc cgtctctatg gtcagggtct ctcctagacc 840

aagccccacc cagcagccga ggccaaaagg gttccccaaa gtgggagagg agtgagagat 900

cccaggagac ctcaacagga ccccacccat aggtacacac aaaaaagggg ggatcgaggc 960

cagacacggt ggctcacgcc tgtaatccca gcagtttggg aagccgaggc gggtggaaca 1020

cttgaggtca ggggtttgag accagcctgg cttgaacctg ggaggcggag gttgcagtga 1080

gccgagattg cgccactgca ctccagcctg ggcgacagag tgagactccg tctcaaaaaa 1140

aacaaaaagc aggaggattg ggagcctgtc agccccatcc tgagaccccg tcctcatttc 1200

tgtaatgatg gatctcgctc ccactttccc ccaagaacct aataaaggct tgtgaagaaa 1260

aagcaaaaaa aaaaaaaaaa aa 1282

<210> 12
<211> 1270
<212> DNA
<213> Homo sapiens

<400> 12
ggaagtctgt caactgggag ggggagaggg gggtgatggg ccaggaatgg ggtccccggg 60

catggtgctg ggcctcctgg tgcagatctg ggccctgcaa gaagcctcaa gcctgagcgt 120

gcagcagggg cccaacttgc tgcaggtgag gcagggcagt caggcgaccc tggtctgcca 180

ggtggaccag gccacagcct gggaacggct ccgtgttaag tggacaaagg atggggccat 240

cctgtgtcaa ccgtacatca ccaacggcag cctcagcctg gggtctgcgg ggccccaggg 300

acggctctcc tggcaggcac ccagccatct caccctgcag ctggaccctg tgagcctcaa 360

ccacagcggg gcgtacgtgt gctgggcggc cgtagagatt cctgagttgg aggaggctga 420

gggcaacata acaaggctct ttgtggaccc agatgacccc acacagaaca gaaaccggat 480

cgcaagcttc ccaggattcc tcttcgtgct gctgggggtg ggaagcatgg gtgtggctgc 540

gatcgtgtgg ggtgcctggt tctggggccg ccgcagctgc cagcaaaggg actcaggaaa 600

tgcattctac agcaacgtcc tataccggcc ccgggggggcc ccaaagaaga gtgaggactg 660

ctctggagag gggaaggacc agagggggcca gagcatttat tcaacctcct tcccgcaacc 720

ggccccccgc cagccgcacc tggcgtcaag accctgcccc agcccgagac cctgccccag 780

ccccaggccc ggccaccccg tctctatggt cagggtctct cctagaccaa gccccaccca 840

gcagccgagg ccaaaagggt tccccaaagt gggagaggag tgagagatcc caggagacct 900

caacaggacc ccacccatag gtacacacaa aaaagggggg atcgaggcca gacacggtgg 960

ctcacgcctg taatcccagc agtttgggaa gccgaggcgg gtggaacact tgaggtcagg 1020

ggtttgagac cagcctggct tgaacctggg aggcggaggt tgcagtgagc cgagattgcg 1080

ccactgcact ccagcctggg cgacagagtg agactccgtc tcaaaaaaaa caaaaagcag 1140

gaggattggg agcctgtcag ccccatcctg agaccccgtc ctcatttctg taatgatgga 1200

tctcgctccc actttccccc aagaacctaa taaaggcttg tgaagaaaaa gcaaaaaaaa 1260

aaaaaaaaaa 1270

<210> 13
<211> 918
<212> DNA
<213> Homo sapiens

<400> 13
ggaagtctgt caactgggag ggggagaggg gggtgatggg ccaggaatgg ggtccccggg 60

catggtgctg ggcctcctgg tgcagatctg ggatgacccc acacagaaca gaaaccggat 120

cgcaagcttc ccaggattcc tcttcgtgct gctgggggtg ggaagcatgg gtgtggctgc 180

gatcgtgtgg ggtgcctggt tctggggccg ccgcagctgc cagcaaaggg actcaggtaa 240

cagcccagga aatgcattct acagcaacgt cctataccgg ccccgggggg ccccaaagaa 300

gagtgaggac tgctctggag aggggaagga ccagaggggc cagagcattt attcaacctc 360

cttcccgcaa ccggcccccc gccagccgca cctggcgtca agaccctgcc ccagcccgag 420

accctgcccc agccccaggc ccggccaccc cgtctctatg gtcagggtct ctcctagacc 480

aagccccacc cagcagccga ggccaaaagg gttccccaaa gtgggagagg agtgagagat 540

cccaggagac ctcaacagga ccccacccat aggtacacac aaaaaagggg ggatcgaggc 600

cagacacggt ggctcacgcc tgtaatccca gcagtttggg aagccgaggc gggtggaaca 660

cttgaggtca ggggtttgag accagcctgg cttgaacctg ggaggcggag gttgcagtga 720

gccgagattg cgccactgca ctccagcctg ggcgacagag tgagactccg tctcaaaaaa 780

aacaaaaagc aggaggattg ggagcctgtc agccccatcc tgagaccccg tcctcatttc 840

tgtaatgatg gatctcgctc ccactttccc ccaagaacct aataaaggct tgtgaagaaa 900

aagcaaaaaa aaaaaaaa 918

<210> 14
<211> 282
<212> PRT
<213> Homo sapiens

<400> 14

Met Gly Ser Pro Gly Met Val Leu Gly Leu Leu Val Gln Ile Trp Ala
1 5 10 15

Leu Gln Glu Ala Ser Ser Leu Ser Val Gln Gln Gly Pro Asn Leu Leu
20 25 30

Gln Val Arg Gln Gly Ser Gln Ala Thr Leu Val Cys Gln Val Asp Gln
35 40 45

Ala Thr Ala Trp Glu Arg Leu Arg Val Lys Trp Thr Lys Asp Gly Ala

```
        50                  55                  60

Ile Leu Cys Gln Pro Tyr Ile Thr Asn Gly Ser Leu Ser Leu Gly Val
65                  70                  75                  80

Cys Gly Pro Gln Gly Arg Leu Ser Trp Gln Ala Pro Ser His Leu Thr
                85                  90                  95

Leu Gln Leu Asp Pro Val Ser Leu Asn His Ser Gly Ala Tyr Val Cys
            100                 105                 110

Trp Ala Ala Val Glu Ile Pro Glu Leu Glu Glu Ala Glu Gly Asn Ile
        115                 120                 125

Thr Arg Leu Phe Val Asp Pro Asp Asp Pro Thr Gln Asn Arg Asn Arg
    130                 135                 140

Ile Ala Ser Phe Pro Gly Phe Leu Phe Val Leu Leu Gly Val Gly Ser
145                 150                 155                 160

Met Gly Val Ala Ala Ile Val Trp Gly Ala Trp Phe Trp Gly Arg Arg
                165                 170                 175

Ser Cys Gln Gln Arg Asp Ser Gly Asn Ser Pro Gly Asn Ala Phe Tyr
            180                 185                 190

Ser Asn Val Leu Tyr Arg Pro Arg Gly Ala Pro Lys Lys Ser Glu Asp
        195                 200                 205

Cys Ser Gly Glu Gly Lys Asp Gln Arg Gly Gln Ser Ile Tyr Ser Thr
    210                 215                 220

Ser Phe Pro Gln Pro Ala Pro Arg Gln Pro His Leu Ala Ser Arg Pro
225                 230                 235                 240

Cys Pro Ser Pro Arg Pro Cys Pro Ser Pro Arg Pro Gly His Pro Val
                245                 250                 255

Ser Met Val Arg Val Ser Pro Arg Pro Ser Pro Thr Gln Gln Pro Arg
            260                 265                 270

Pro Lys Gly Phe Pro Lys Val Gly Glu Glu
        275                 280
```

<210>  15
<211>  278
<212>  PRT
<213>  Homo sapiens

<400> 15

Met Gly Ser Pro Gly Met Val Leu Gly Leu Leu Val Gln Ile Trp Ala
1               5                   10                  15

Leu Gln Glu Ala Ser Ser Leu Ser Val Gln Gln Gly Pro Asn Leu Leu
            20                  25                  30

Gln Val Arg Gln Gly Ser Gln Ala Thr Leu Val Cys Gln Val Asp Gln
        35                  40                  45

Ala Thr Ala Trp Glu Arg Leu Arg Val Lys Trp Thr Lys Asp Gly Ala
    50                  55                  60

Ile Leu Cys Gln Pro Tyr Ile Thr Asn Gly Ser Leu Ser Leu Gly Val
65                  70                  75                  80

Cys Gly Pro Gln Gly Arg Leu Ser Trp Gln Ala Pro Ser His Leu Thr
                85                  90                  95

Leu Gln Leu Asp Pro Val Ser Leu Asn His Ser Gly Ala Tyr Val Cys
            100                 105                 110

Trp Ala Ala Val Glu Ile Pro Glu Leu Glu Glu Ala Glu Gly Asn Ile
        115                 120                 125

Thr Arg Leu Phe Val Asp Pro Asp Asp Pro Thr Gln Asn Arg Asn Arg
    130                 135                 140

Ile Ala Ser Phe Pro Gly Phe Leu Phe Val Leu Leu Gly Val Gly Ser
145                 150                 155                 160

Met Gly Val Ala Ala Ile Val Trp Gly Ala Trp Phe Trp Gly Arg Arg
                165                 170                 175

Ser Cys Gln Gln Arg Asp Ser Gly Asn Ala Phe Tyr Ser Asn Val Leu
            180                 185                 190

Tyr Arg Pro Arg Gly Ala Pro Lys Lys Ser Glu Asp Cys Ser Gly Glu
        195                 200                 205

Gly Lys Asp Gln Arg Gly Gln Ser Ile Tyr Ser Thr Ser Phe Pro Gln
    210                 215                 220

Pro Ala Pro Arg Gln Pro His Leu Ala Ser Arg Pro Cys Pro Ser Pro
225                 230                 235                 240

Arg Pro Cys Pro Ser Pro Arg Pro Gly His Pro Val Ser Met Val Arg
245 250 255

Val Ser Pro Arg Pro Ser Pro Thr Gln Gln Pro Arg Pro Lys Gly Phe
260 265 270

Pro Lys Val Gly Glu Glu
275

<210> 16
<211> 162
<212> PRT
<213> Homo sapiens

<400> 16

Met Gly Ser Pro Gly Met Val Leu Gly Leu Leu Val Gln Ile Trp Asp
1 5 10 15

Asp Pro Thr Gln Asn Arg Asn Arg Ile Ala Ser Phe Pro Gly Phe Leu
20 25 30

Phe Val Leu Leu Gly Val Gly Ser Met Gly Val Ala Ala Ile Val Trp
35 40 45

Gly Ala Trp Phe Trp Gly Arg Arg Ser Cys Gln Gln Arg Asp Ser Gly
50 55 60

Asn Ser Pro Gly Asn Ala Phe Tyr Ser Asn Val Leu Tyr Arg Pro Arg
65 70 75 80

Gly Ala Pro Lys Lys Ser Glu Asp Cys Ser Gly Glu Gly Lys Asp Gln
85 90 95

Arg Gly Gln Ser Ile Tyr Ser Thr Ser Phe Pro Gln Pro Ala Pro Arg
100 105 110

Gln Pro His Leu Ala Ser Arg Pro Cys Pro Ser Pro Arg Pro Cys Pro
115 120 125

Ser Pro Arg Pro Gly His Pro Val Ser Met Val Arg Val Ser Pro Arg
130 135 140

Pro Ser Pro Thr Gln Gln Pro Arg Pro Lys Gly Phe Pro Lys Val Gly
145 150 155 160

Glu Glu

<210> 17
<211> 2734
<212> DNA
<213> Homo sapiens

<400> 17

```
ctggtaatct gtggcgtaac aagacctcca ggtgattctc tgtattagca gccgctctgt      60
gctccctgat tcctccagaa aagcacaagg atttaatcca gatagatata aatttcacct     120
gggatcattt atcattccac attcttcttt aagaaatgtg ctaaagagcc acagatgggt     180
cttgatgaaa acaaaggaaa agccatgaag tttctacagc ataattagca tcatcattat     240
tctggctgga gcaattgcac tcatcattgg ctttggtatt tcagggagac actccatcac     300
agtcactact gtcgcctcag ctgggaacat tggggaggat ggaatcctga gctgcacttt     360
tgaacctgac atcaaacttt ctgatatcgt gatacaatgg ctgaaggaag gtgttttagg     420
cttggtccat gagttcaaag aaggcaaaga tgagctgtcg gagcaggatg aaatgttcag     480
aggccggaca gcagtgtttg ctgatcaagt gatagttggc aatgcctctt tgcggctgaa     540
aaacgtgcaa ctcacagatg ctggcaccta caaatgttat atcatcactt ctaaaggcaa     600
ggggaatgct aaccttgagt ataaaactgg agccttcagc atgccggaag tgaatgtgga     660
ctataatgcc agctcagaga ccttgcggtg tgaggctccc cgatggttcc cccagcccac     720
agtggtctgg gcatcccaag ttgaccaggg agccaacttc tcggaagtct ccaataccag     780
ctttgagctg aactctgaga atgtgaccat gaaggttgtg tctgtgctct acaatgttac     840
gatcaacaac acatactcct gtatgattga aaatgacatt gccaaagcaa caggggatat     900
caaagtgaca gaatcggaga tcaaaaggcg gagtcaccta cagctgctaa actcaaaggc     960
ttctctgtgt gtctcttctt tctttgccat cagctgggca cttctgcctc tcagccctta    1020
cctgatgcta aaataatgtg cctcggccac aaaaaagcat gcaaagtcat tgttacaaca    1080
gggatctaca gaactatttc accaccagat atgacctagt tttatatttc tgggaggaaa    1140
tgaattcata tctagaagtc tggagtgagc aaacaagagc aagaaacaaa aagaagccaa    1200
aagcagaagg ctccaatatg aacaagataa atctatcttc aaagacatat tagaagttgg    1260
gaaaataatt catgtgaact agacaagtgt gttaagagtg ataagtaaaa tgcacgtgga    1320
gacaagtgca tccccagatc tcagggacct ccccctgcct gtcacctggg gagtgagagg    1380
acaggatagt gcatgttctt tgtctctgaa tttttagtta tatgtgctgt aatgttgctc    1440
tgaggaagcc cctggaaagt ctatcccaac atatccacat cttatattcc acaaattaag    1500
ctgtagtatg taccctaaga cgctgctaat tgactgccac ttcgcaactc aggggcggct    1560
gcattttagt aatgggtcaa atgattcact tttatgatg cttccaaagg tgccttggct    1620
tctcttccca actgacaaat gccaaagttg agaaaaatga tcataatttt agcataaaca    1680
gagcagtcgg cgacaccgat tttataaata aactgagcac cttcttttta aacaaacaaa    1740
```

tgcgggttta tttctcagat gatgttcatc cgtgaatggt ccagggaagg acctttcacc 1800

ttgtctatat ggcattatgt catcacaagc tctgaggctt ctcctttcca tcctgcgtgg 1860

acagctaaga cctcagtttt caatagcatc tagagcagtg ggactcagct ggggtgattt 1920

cgccccccat ctccggggga atgtctgaag acaattttgg ttacctcaat gagggagtgg 1980

aggaggatac agtgctacta ccaactagtg gatagaggcc agggatgctg ctcaacctcc 2040

taccatgtac aggacgtctc cccattacaa ctacccaatc cgaagtgtca actgtgtcag 2100

ggctaagaaa ccctggtttt gagtagaaaa gggcctggaa agaggggagc caacaaatct 2160

gtctgcttcc tcacattagt cattggcaaa taagcattct gtctctttgg ctgctgcctc 2220

agcacagaga gccagaactc tatcgggcac caggataaca tctctcagtg aacagagttg 2280

acaaggccta tgggaaatgc ctgatgggat tatcttcagc ttgttgagct tctaagtttc 2340

tttcccttca ttctaccctg caagccaagt tctgtaagag aaatgcctga gttctagctc 2400

aggttttctt actctgaatt tagatctcca gaccctgcct ggccacaatt caaattaagg 2460

caacaaacat ataccttcca tgaagcacac acagactttt gaaagcaagg acaatgactg 2520

cttgaattga ggccttgagg aatgaagctt tgaaggaaaa gaatactttg tttccagccc 2580

ccttcccaca ctcttcatgt gttaaccact gccttcctgg accttggagc cacggtgact 2640

gtattacatg ttgttataga aaactgattt tagagttctg atcgttcaag agaatgatta 2700

aatatacatt tcctacacca aaaaaaaaaa aaaa 2734

<210> 18
<211> 2605
<212> DNA
<213> Homo sapiens

<400> 18
gtgagtcacc aaggaaggca gcggcagctc cactcagcca gtacccagat acgctgggaa 60

ccttccccag ccatggcttc cctggggcag atcctcttct ggagcataat tagcatcatc 120

attattctgg ctggagcaat tgcactcatc attggctttg gtatttcagg gagacactcc 180

atcacagtca ctactgtcgc ctcagctggg aacattgggg aggatggaat cctgagctgc 240

acttttgaac ctgacatcaa actttctgat atcgtgatac aatggctgaa ggaaggtgtt 300

ttaggcttgg tccatgagtt caaagaaggc aaagatgagc tgtcggagca ggatgaaatg 360

ttcagaggcc ggacagcagt gtttgctgat caagtgatag ttggcaatgc ctctttgcgg 420

ctgaaaaacg tgcaactcac agatgctggc acctacaaat gttatatcat cacttctaaa 480

ggcaagggga atgctaacct tgagtataaa actggagcct tcagcatgcc ggaagtgaat 540

gtggactata atgccagctc agagaccttg cggtgtgagg ctccccgatg gttcccccag 600

cccacagtgg tctgggcatc ccaagttgac cagggagcca acttctcgga agtctccaat 660

accagctttg agctgaactc tgagaatgtg accatgaagg ttgtgtctgt gctctacaat 720
gttacgatca acaacacata ctcctgtatg attgaaaatg acattgccaa agcaacaggg 780
gatatcaaag tgacagaatc ggagatcaaa aggcggagtc acctacagct gctaaactca 840
aaggcttctc tgtgtgtctc ttctttcttt gccatcagct gggcacttct gcctctcagc 900
ccttacctga tgctaaaata atgtgcctcg gccacaaaaa agcatgcaaa gtcattgtta 960
caacagggat ctacagaact atttcaccac cagatatgac ctagttttat atttctggga 1020
ggaaatgaat tcatatctag aagtctggag tgagcaaaca agagcaagaa acaaaaagaa 1080
gccaaaagca gaaggctcca atatgaacaa gataaatcta tcttcaaaga catattagaa 1140
gttgggaaaa taattcatgt gaactagaca agtgtgttaa gagtgataag taaaatgcac 1200
gtggagacaa gtgcatcccc agatctcagg gacctccccc tgcctgtcac ctggggagtg 1260
agaggacagg atagtgcatg ttctttgtct ctgaattttt agttatatgt gctgtaatgt 1320
tgctctgagg aagcccctgg aaagtctatc ccaacatatc cacatcttat attccacaaa 1380
ttaagctgta gtatgtaccc taagacgctg ctaattgact gccacttcgc aactcagggg 1440
cggctgcatt ttagtaatgg gtcaaatgat tcacttttta tgatgcttcc aaaggtgcct 1500
tggcttctct tcccaactga caaatgccaa agttgagaaa aatgatcata attttagcat 1560
aaacagagca gtcggcgaca ccgattttat aaataaactg agcaccttct ttttaaacaa 1620
acaaatgcgg gtttatttct cagatgatgt tcatccgtga atggtccagg gaaggacctt 1680
tcaccttgtc tatatggcat tatgtcatca caagctctga ggcttctcct ttccatcctg 1740
cgtggacagc taagacctca gttttcaata gcatctagag cagtgggact cagctggggt 1800
gatttcgccc cccatctccg ggggaatgtc tgaagacaat tttggttacc tcaatgaggg 1860
agtggaggag gatacagtgc tactaccaac tagtggatag aggccaggga tgctgctcaa 1920
cctcctacca tgtacaggac gtctccccat tacaactacc caatccgaag tgtcaactgt 1980
gtcagggcta agaaaccctg gttttgagta gaaaagggcc tggaaagagg ggagccaaca 2040
aatctgtctg cttcctcaca ttagtcattg gcaaataagc attctgtctc tttggctgct 2100
gcctcagcac agagagccag aactctatcg ggcaccagga taacatctct cagtgaacag 2160
agttgacaag gcctatggga aatgcctgat gggattatct tcagcttgtt gagcttctaa 2220
gtttctttcc cttcattcta ccctgcaagc caagttctgt aagagaaatg cctgagttct 2280
agctcaggtt ttcttactct gaatttagat ctccagaccc tgcctggcca caattcaaat 2340
taaggcaaca aacatatacc ttccatgaag cacacacaga cttttgaaag caaggacaat 2400
gactgcttga attgaggcct tgaggaatga agctttgaag gaaaagaata ctttgtttcc 2460
agccccccttc ccacactctt catgtgttaa ccactgcctt cctggacctt ggagccacgg 2520

tgactgtatt acatgttgtt atagaaaact gattttagag ttctgatcgt tcaagagaat 2580

gattaaatat acatttccta cacca 2605

<210> 19
<211> 2304
<212> DNA
<213> Homo sapiens

<400> 19
acactattta aggccaatac acgggagctg gttgtgagtc accaaggaag gcagcggcag 60

ctccactcag ccagtaccca gatacgctgg gaaccttccc cagccatggc ttccctgggg 120

cagatcctct tctggagcat aattagcatc atcattattc tggctggagc aattgcactc 180

atcattggct ttggtatttc agccttcagc atgccggaag tgaatgtgga ctataatgcc 240

agctcagaga ccttgcggtg tgaggctccc cgatggttcc cccagcccac agtggtctgg 300

gcatcccaag ttgaccaggg agccaacttc tcggaagtct ccaataccag ctttgagctg 360

aactctgaga atgtgaccat gaaggttgtg tctgtgctct acaatgttac gatcaacaac 420

acatactcct gtatgattga aaatgacatt gccaaagcaa caggggatat caaagtgaca 480

gaatcggaga tcaaaaggcg gagtcaccta cagctgctaa actcaaaggc ttctctgtgt 540

gtctcttctt tctttgccat cagctgggca cttctgcctc tcagccctta cctgatgcta 600

aaataatgtg cctcggccac aaaaaagcat gcaaagtcat tgttacaaca gggatctaca 660

gaactatttc accaccagat atgacctagt tttatatttc tgggaggaaa tgaattcata 720

tctagaagtc tggagtgagc aaacaagagc aagaaacaaa aagaagccaa aagcagaagg 780

ctccaatatg aacaagataa atctatcttc aaagacatat tagaagttgg gaaaataatt 840

catgtgaact agacaagtgt gttaagagtg ataagtaaaa tgcacgtgga gacaagtgca 900

tccccagatc tcagggacct ccccctgcct gtcacctggg gagtgagagg acaggatagt 960

gcatgttctt tgtctctgaa tttttagtta tatgtgctgt aatgttgctc tgaggaagcc 1020

cctggaaagt ctatcccaac atatccacat cttatattcc acaaattaag ctgtagtatg 1080

taccctaaga cgctgctaat tgactgccac ttcgcaactc aggggcggct gcattttagt 1140

aatgggtcaa atgattcact tttatgatg cttccaaagg tgccttggct tctcttccca 1200

actgacaaat gccaaagttg agaaaaatga tcataatttt agcataaaca gagcagtcgg 1260

cgacaccgat tttataaata aactgagcac cttcttttta aacaaacaaa tgcgggttta 1320

tttctcagat gatgttcatc cgtgaatggt ccagggaagg acctttcacc ttgtctatat 1380

ggcattatgt catcacaagc tctgaggctt ctcctttcca tcctgcgtgg acagctaaga 1440

cctcagtttt caatagcatc tagagcagtg ggactcagct ggggtgattt cgcccccat 1500

ctccgggga atgtctgaag acaattttgg ttacctcaat gagggagtgg aggaggatac 1560

agtgctacta ccaactagtg gatagaggcc agggatgctg ctcaacctcc taccatgtac 1620

aggacgtctc cccattacaa ctacccaatc cgaagtgtca actgtgtcag ggctaagaaa 1680

ccctggtttt gagtagaaaa gggcctggaa agaggggagc caacaaatct gtctgcttcc 1740

tcacattagt cattggcaaa taagcattct gtctctttgg ctgctgcctc agcacagaga 1800

gccagaactc tatcgggcac caggataaca tctctcagtg aacagagttg acaaggccta 1860

tgggaaatgc ctgatgggat tatcttcagc ttgttgagct tctaagtttc tttcccttca 1920

ttctaccctg caagccaagt tctgtaagag aaatgcctga gttctagctc aggttttctt 1980

actctgaatt tagatctcca gaccctgcct ggccacaatt caaattaagg caacaaacat 2040

ataccttcca tgaagcacac acagactttt gaaagcaagg acaatgactg cttgaattga 2100

ggccttgagg aatgaagctt tgaaggaaaa gaatactttg tttccagccc ccttcccaca 2160

ctcttcatgt gttaaccact gccttcctgg accttggagc cacggtgact gtattacatg 2220

ttgttataga aaactgattt tagagttctg atcgttcaag agaatgatta aatatacatt 2280

tcctacacca aaaaaaaaaa aaaa 2304

<210> 20
<211> 187
<212> PRT
<213> Homo sapiens

<400> 20

Met Phe Arg Gly Arg Thr Ala Val Phe Ala Asp Gln Val Ile Val Gly
1 5 10 15

Asn Ala Ser Leu Arg Leu Lys Asn Val Gln Leu Thr Asp Ala Gly Thr
20 25 30

Tyr Lys Cys Tyr Ile Ile Thr Ser Lys Gly Lys Gly Asn Ala Asn Leu
35 40 45

Glu Tyr Lys Thr Gly Ala Phe Ser Met Pro Glu Val Asn Val Asp Tyr
50 55 60

Asn Ala Ser Ser Glu Thr Leu Arg Cys Glu Ala Pro Arg Trp Phe Pro
65 70 75 80

Gln Pro Thr Val Val Trp Ala Ser Gln Val Asp Gln Gly Ala Asn Phe
85 90 95

Ser Glu Val Ser Asn Thr Ser Phe Glu Leu Asn Ser Glu Asn Val Thr
100 105 110

Met Lys Val Val Ser Val Leu Tyr Asn Val Thr Ile Asn Asn Thr Tyr

115 120 125

Ser Cys Met Ile Glu Asn Asp Ile Ala Lys Ala Thr Gly Asp Ile Lys
130 135 140

Val Thr Glu Ser Glu Ile Lys Arg Arg Ser His Leu Gln Leu Leu Asn
145 150 155 160

Ser Lys Ala Ser Leu Cys Val Ser Ser Phe Phe Ala Ile Ser Trp Ala
165 170 175

Leu Leu Pro Leu Ser Pro Tyr Leu Met Leu Lys
180 185

<210> 21
<211> 282
<212> PRT
<213> Homo sapiens

<400> 21

Met Ala Ser Leu Gly Gln Ile Leu Phe Trp Ser Ile Ile Ser Ile Ile
1 5 10 15

Ile Ile Leu Ala Gly Ala Ile Ala Leu Ile Ile Gly Phe Gly Ile Ser
20 25 30

Gly Arg His Ser Ile Thr Val Thr Thr Val Ala Ser Ala Gly Asn Ile
35 40 45

Gly Glu Asp Gly Ile Leu Ser Cys Thr Phe Glu Pro Asp Ile Lys Leu
50 55 60

Ser Asp Ile Val Ile Gln Trp Leu Lys Glu Gly Val Leu Gly Leu Val
65 70 75 80

His Glu Phe Lys Glu Gly Lys Asp Glu Leu Ser Glu Gln Asp Glu Met
85 90 95

Phe Arg Gly Arg Thr Ala Val Phe Ala Asp Gln Val Ile Val Gly Asn
100 105 110

Ala Ser Leu Arg Leu Lys Asn Val Gln Leu Thr Asp Ala Gly Thr Tyr
115 120 125

Lys Cys Tyr Ile Ile Thr Ser Lys Gly Lys Gly Asn Ala Asn Leu Glu
130 135 140

Tyr Lys Thr Gly Ala Phe Ser Met Pro Glu Val Asn Val Asp Tyr Asn

145 150 155 160

Ala Ser Ser Glu Thr Leu Arg Cys Glu Ala Pro Arg Trp Phe Pro Gln
165 170 175

Pro Thr Val Val Trp Ala Ser Gln Val Asp Gln Gly Ala Asn Phe Ser
180 185 190

Glu Val Ser Asn Thr Ser Phe Glu Leu Asn Ser Glu Asn Val Thr Met
195 200 205

Lys Val Val Ser Val Leu Tyr Asn Val Thr Ile Asn Asn Thr Tyr Ser
210 215 220

Cys Met Ile Glu Asn Asp Ile Ala Lys Ala Thr Gly Asp Ile Lys Val
225 230 235 240

Thr Glu Ser Glu Ile Lys Arg Arg Ser His Leu Gln Leu Leu Asn Ser
245 250 255

Lys Ala Ser Leu Cys Val Ser Ser Phe Phe Ala Ile Ser Trp Ala Leu
260 265 270

Leu Pro Leu Ser Pro Tyr Leu Met Leu Lys
275 280

<210> 22
<211> 166
<212> PRT
<213> Homo sapiens

<400> 22

Met Ala Ser Leu Gly Gln Ile Leu Phe Trp Ser Ile Ile Ser Ile Ile
1 5 10 15

Ile Ile Leu Ala Gly Ala Ile Ala Leu Ile Ile Gly Phe Gly Ile Ser
20 25 30

Ala Phe Ser Met Pro Glu Val Asn Val Asp Tyr Asn Ala Ser Ser Glu
35 40 45

Thr Leu Arg Cys Glu Ala Pro Arg Trp Phe Pro Gln Pro Thr Val Val
50 55 60

Trp Ala Ser Gln Val Asp Gln Gly Ala Asn Phe Ser Glu Val Ser Asn
65 70 75 80

Thr Ser Phe Glu Leu Asn Ser Glu Asn Val Thr Met Lys Val Val Ser

```
                85                  90                  95

Val Leu Tyr Asn Val Thr Ile Asn Asn Thr Tyr Ser Cys Met Ile Glu
            100                 105                 110

Asn Asp Ile Ala Lys Ala Thr Gly Asp Ile Lys Val Thr Glu Ser Glu
        115                 120                 125

Ile Lys Arg Arg Ser His Leu Gln Leu Leu Asn Ser Lys Ala Ser Leu
    130                 135                 140

Cys Val Ser Ser Phe Phe Ala Ile Ser Trp Ala Leu Leu Pro Leu Ser
145                 150                 155                 160

Pro Tyr Leu Met Leu Lys
                165
```